# EUROPEAN PATENT APPLICATION

(11) **EP 4 223 129 A1**
(43) Date of publication of application: **09.08.2023**
(21) Application number: 21875363.0
(22) Date of filing: 22.09.2021

(54) **MUTANT TRANSGLUTAMINASE**

(30) Priority: 29.09.2020 JP 2020163299
(71) Applicant: AJINOMOTO CO., INC., Chuo-ku Tokyo 104-8315 (JP)
(72) Inventor: TAKAHASHI, Kazutoshi, Kawasaki-shi, Kanagawa 210-8681 (JP); ONO, Takuto, Kawasaki-shi, Kanagawa 210-8681 (JP); ARAKI, Nao, Kawasaki-shi, Kanagawa 210-8681 (JP); KOBAYASHI, Hiroaki, Kawasaki-shi, Kanagawa 210-8681 (JP)
(74) Representative: Strehl Schübel-Hopf & Partner
(86) International application number: PCT/JP2021/034780
(87) International publication number: WO 2022/071061

(57) **Abstract**

A mutant transglutaminase (mutant TG) having high thermostability and/or high pH stability is provided. A mutant TG having a mutation at amino acid residue(s) such as D1, Y24, R48, S101, G102, N139, D142, L147, K152, G157, R167, N176, K181, E182, H188, D189, R208, T245, S246, G250, G275, S284, H289, G301, and K327 and/or a mutation of introducing a disulfide bond.

## Description

### Technical Field

The present invention relates to a mutant transglutaminase (mutant TG) having high thermostability and/or high pH stability and use of the same.

### Background Art

Transglutaminase (TG) is known as an enzyme that catalyzes cross-linking of proteins, and has been used for improving physical properties of foods.

Various mutant TGs having modified functions, such as mutant TGs having improved thermostability and mutant TGs having improved pH stability, have also been known. As the mutant TGs having improved thermostability, for example, mutant TGs disclosed in Patent documents 1 to 4 have been known. As the mutant TGs having improved pH stability, for example, mutant TGs disclosed in Patent document 4 have been known.

In addition, Patent document 1 discloses mutations including G275A as mutations of making TG into a deamidase.

### Prior art references

### Patent documents

Patent document 1: WO2019/107288A1
Patent document 2: WO2010/101256A1
Patent document 3: WO2008/099898A1
Patent document 4: WO2019/094301A1

### Summary of Invention

### Object to be Achieved by the Invention

An object of the present invention is to provide a mutant TG having high thermostability and/or high pH stability.

### Means for Achieving the Object

The inventors of the present invention found mutations for improving thermostability and/or pH stability of TGs and accomplished the present invention.

The present invention can be thus embodied as follows.
[1] A method for producing a food, the method comprising:
   a step of treating a food raw material with a mutant transglutaminase under heating conditions, acidic condition, or alkaline conditions,
   wherein the mutant transglutaminase is a protein having a specific mutation in the amino acid sequence of a wild-type transglutaminase, and having a transglutaminase activity, and
   wherein the specific mutation is a mutation of improving thermostability and/or pH stability.
[2] The method mentioned above, wherein the specific mutation comprises the mutation (A) and/or the mutation (B) shown below:
   the mutation (A): mutation(s) at one or more amino acid residues selected from the followings:
   G275, D1, Y24, R48, S101, G102, N139, D142, L147, K152, G157, R167, N176, K181, E182, H188, D189, R208, T245, S246, G250, S284, H289, G301, and K327;
   the mutation (B): a mutation of introducing a disulfide bond.
[3] The method mentioned above, wherein the mutation (A) comprises mutation(s) at one or more amino acid residues selected from the followings:
   G275, S101, G157, R208, and G250.
[4] The method mentioned above, wherein the mutation (A) comprises one or more mutations selected from the followings:
   G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.
[5] The method mentioned above, wherein the mutation (A) comprises one or more mutations selected from the followings:
   G275A, S101P, G157(A, R, S), R208E, and G250(N, R, S).
[6] The method mentioned above, wherein the mutation (A) comprises any one of the following mutations:
   S101P/G157(A, R, S)/R208E/G250(N, R, S)/G275A, S101P/G157(A, R, S)/G250(N, R, S), and S101P/G157(A, R, S)/R208E/G250(N, R, S).
[7] The method mentioned above, wherein the mutation (A) comprises any one of the following mutations:
   S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.
[8] The method mentioned above, wherein the mutation (A) comprises any one of the following mutations:
   S101P/G157R/R208E/G250S/G275A and
   S101P/G157S/R208E/G250R/G275A.
[9] The method mentioned above, wherein the mutation (B) comprises one or more mutations selected from the followings:
   D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.
[10] The method mentioned above, wherein the mutation (B) is mutations of introducing two or more disulfide bonds.
[11] The method mentioned above, wherein the mutation (B) is mutations of introducing three or more disulfide bonds.
[12] The method mentioned above, wherein the mutation (B) comprises D3C/G283C.
[13] The method mentioned above, wherein the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.
[14] The method mentioned above, wherein the mutation (B) comprises any one of the following mutations:
   D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.
[15] The method mentioned above, wherein the specific mutation comprises the mutation (A) and the mutation (B).
[16] The method mentioned above, wherein the residual activity after heat-treating the mutant transglutaminase at 65°C, pH6.0 for 10 minutes is 15% or more.
[17] The method mentioned above, wherein the residual activity after heat-treating the mutant transglutaminase at 75°C, pH6.0 for 10 minutes is 10% or more.
[18] The method mentioned above, wherein the residual activity after treating the mutant transglutaminase at pH4.0, 37°C for four hours is 60% or more in term of the relative value to the residual activity after treating the mutant TG at pH6.0, 37°C for four hours which is taken as 100%.
[19] The method mentioned above, wherein the wild-type transglutaminase is a protein having an amino acid sequence of a mature transglutaminase of a *Streptomyces* bacterium.
[20] The method mentioned above, wherein the *Streptomyces* bacterium is *Streptomyces mobaraensis.*
[21] The method mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[22] The method mentioned above, which satisfies any one of (1) to (3) shown below:
   (1) the step is carried out under the heating conditions, and the food is a milk processed food, egg processed food, plant processed food, meat processed food, seafood processed food, or gelatin processed food;
   (2) the step is carried out under the acidic conditions, and the food is a cereal, potato, bean, nut, meat, seafood, dairy product, fat or oil, seasoning, beverage, fruit juice, or a processed product thereof;
   (3) the step is carried out under the alkaline conditions, and the food is a plant beverage, a seaweed extract, or a processed product thereof.
[23] The method mentioned above, which satisfies any one of (1) to (3) shown below:
   (1) the step is carried out under the heating conditions, and the food is processed cheese, pre-incubated yogurt, egg omelet, steamed egg custard, egg tofu, tofu, yuba, noodle, bread, ham, sausage, hamburger steak, ice cream, wheat dough, jelly, gummy, margarine, or paste product;
   (2) the step is carried out under the acidic conditions, and the food is white rice, brown rice, vinegared rice, oatmeal, rice flour, wheat flour, buckwheat flour, bran, rice cake, gyuhi, bread, noodle, fu, buckwheat noodle, white potato, soy milk, chocolate, fried tofu, pea protein, meat, gelatin, shrimp, crab, adductor, herring roe, yogurt, butter, cheese, ice cream, mayonnaise, ketchup, mustard, soy sauce, miso, coffee-based drink, nutritional drink, fruit juice drink, cocoa, beer, sake lee, fruit juice gummy, or a processed product thereof;
   (3) the step is carried out under the alkaline conditions, and the food is soy milk, kelp soup stock, or a processed product thereof.
[24] The method mentioned above, wherein a heating temperature during the step is 60°C or higher.
[25] A composition for producing a food, the composition comprising a mutant transglutaminase,
   wherein the food is a food that is heated during production or a food having low pH or high pH,
   wherein the mutant transglutaminase is a protein having a specific mutation in the amino acid sequence of a wild-type transglutaminase, and having a transglutaminase activity, and
   wherein the specific mutation is a mutation of improving thermostability and/or pH stability.
[26] The composition mentioned above, wherein the specific mutation comprises the mutation (A) and/or the mutation (B) shown below:
   the mutation (A): mutation(s) at one or more amino acid residues selected from the followings:
   G275, D1, Y24, R48, S101, G102, N139, D142, L147, K152, G157, R167, N176, K181, E182, H188, D189, R208, T245, S246, G250, S284, H289, G301, and K327;
   the mutation (B): a mutation of introducing a disulfide bond.
[27] The composition mentioned above, wherein the mutation (A) comprises mutation(s) at one or more amino acid residues selected from the followings:
   G275, S101, G157, R208, and G250.
[28] The composition mentioned above, wherein the mutation (A) comprises one or more mutations selected from the followings:
   G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.
[29] The composition mentioned above, wherein the mutation (A) comprises one or more mutations selected from the followings:
   G275A, S101P, G157(A, R, S), R208E, and G250(N, R, S).
[30] The composition mentioned above, wherein the mutation (A) comprises any one of the following mutations:
   S101P/G157(A, R, S)/R208E/G250(N, R, S)/G275A, S101P/G157(A, R, S)/G250(N, R, S), and S101P/G157(A, R, S)/R208E/G250(N, R, S).
[31] The composition mentioned above, wherein the mutation (A) comprises any one of the following mutations:
   S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.
[32] The composition mentioned above, wherein the mutation (A) comprises any one of the following mutations:
   S101P/G157R/R208E/G250S/G275A and
   S101P/G157S/R208E/G250R/G275A.
[33] The composition mentioned above, wherein the mutation (B) comprises one or more mutations selected from the followings:
   D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.
[34] The composition mentioned above, wherein the mutation (B) is mutations of introducing two or more disulfide bonds.
[35] The composition mentioned above, wherein the mutation (B) is mutations of introducing three or more disulfide bonds.
[36] The composition mentioned above, wherein the mutation (B) comprises D3C/G283C.
[37] The composition mentioned above, wherein the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.
[38] The composition mentioned above, wherein the mutation (B) comprises any one of the following mutations:
   D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.
[39] The composition mentioned above, wherein the specific mutation comprises the mutation (A) and the mutation (B).
[40] The composition mentioned above, wherein the residual activity after heat-treating the mutant transglutaminase at 65°C, pH6.0 for 10 minutes is 15% or more.
[41] The composition mentioned above, wherein the residual activity after heat-treating the mutant transglutaminase at 75°C, pH6.0 for 10 minutes is 10% or more.
[42] The composition mentioned above, wherein the residual activity after treating the mutant transglutaminase at pH4.0, 37°C for four hours is 60% or more in term of the relative value to the residual activity after treating the mutant TG at pH6.0, 37°C for four hours which is taken as 100%.
[43] The composition mentioned above, wherein the wild-type transglutaminase is a protein having an amino acid sequence of a mature transglutaminase of a *Streptomyces* bacterium.
[44] The composition mentioned above, wherein the *Streptomyces* bacterium is *Streptomyces mobaraensis.*
[45] The composition mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[46] The composition mentioned above, which satisfies any one of (1) to (3) shown below:
   (1) the step is carried out under the heating conditions, and the food is a milk processed food, egg processed food, plant processed food, meat processed food, seafood processed food, or gelatin processed food;
   (2) the step is carried out under the acidic conditions, and the food is a cereal, potato, bean, nut, meat, seafood, dairy product, fat or oil, seasoning, beverage, fruit juice, or a processed product thereof;
   (3) the step is carried out under the alkaline conditions, and the food is a plant beverage, a seaweed extract, or a processed product thereof.
[47] The composition mentioned above, which satisfies any one of (1) to (3) shown below:
   (1) the step is carried out under the heating conditions, and the food is processed cheese, pre-incubated yogurt, egg omelet, steamed egg custard, egg tofu, tofu, yuba, noodle, bread, ham, sausage, hamburger steak, ice cream, wheat dough, jelly, gummy, margarine, or paste product;
   (2) the step is carried out under the acidic conditions, and the food is white rice, brown rice, vinegared rice, oatmeal, rice flour, wheat flour, buckwheat flour, bran, rice cake, gyuhi, bread, noodle, fu, buckwheat noodle, white potato, soy milk, chocolate, fried tofu, pea protein, meat, gelatin, shrimp, crab, adductor, herring roe, yogurt, butter, cheese, ice cream, mayonnaise, ketchup, mustard, soy sauce, miso, coffee-based drink, nutritional drink, fruit juice drink, cocoa, beer, sake lee, fruit juice gummy, or a processed product thereof;
   (3) the step is carried out under the alkaline conditions, and the food is soy milk, kelp soup stock, or a processed product thereof.
[48] The composition mentioned above, wherein a heating temperature during the production is 60°C or higher.
[49] A mutant transglutaminase, having a specific mutation in the amino acid sequence of a wild-type transglutaminase, and having a transglutaminase activity,
   wherein the specific mutation is a mutation of improving thermostability and/or pH stability,
   wherein the specific mutation comprises the mutation (A) and/or the mutation (B) shown below:
      the mutation (A): mutation(s) at one or more amino acid residues selected from the followings:
      G275, D1, Y24, R48, S101, G102, N139, D142, L147, K152, G157, R167, N176, K181, E182, H188, D189, R208, T245, S246, G250, S284, H289, G301, and K327;
      the mutation (B): a mutation of introducing a disulfide bond, and
      wherein the mutant transglutaminase satisfies (B1) and/or (A1) shown below: (A1) the specific mutation comprises the mutation (A), and the mutation (A) comprises mutation(s) at one or more amino acid residues selected from the followings:
      D1, R48, G102, N139, D142, L147, K152, R167, N176, K181, E182, D189, S246, H289, G301, and K327,
      provided that the mutation at R48 is R48I when the mutation at R48 is solely selected, and
      provided that the mutation at S246 is S246N when the mutation at S246 is solely selected;
         (B1) the specific mutation comprises the mutation (B), and the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.
[50] The mutant transglutaminase mentioned above, wherein the mutation (A) comprises mutation(s) at one or more amino acid residues selected from the followings:
   G275, S101, G157, R208, and G250.
[51] The mutant transglutaminase mentioned above, wherein the mutation (A) comprises one or more mutations selected from the followings:
   G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.
[52] The mutant transglutaminase mentioned above, wherein the mutation (A) comprises one or more mutations selected from the followings:
   G275A, S101P, G157(A, R, S), R208E, and G250(N, R, S).
[53] The mutant transglutaminase mentioned above, wherein the mutation (A) comprises any one of the following mutations:
   S101P/G157(A, R, S)/R208E/G250(N, R, S)/G275A, S101P/G157(A, R, S)/G250(N, R, S), and S101P/G157(A, R, S)/R208E/G250(N, R, S).
[54] The mutant transglutaminase mentioned above, wherein the mutation (A) comprises any one of the following mutations:
   S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.
[55] The mutant transglutaminase mentioned above, wherein the mutation (A) comprises any one of the following mutations:
   S101P/G157R/R208E/G250S/G275 A and
   S101P/G157S/R208E/G250R/G275A.
[56] The mutant transglutaminase mentioned above, wherein the mutation (B) comprises one or more mutations selected from the followings:
   D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.
[57] The mutant transglutaminase mentioned above, wherein the mutation (B) is three or more mutations of introducing disulfide bonds.
[58] The mutant transglutaminase mentioned above, wherein the mutation (B) comprises any one of the following mutations:
   D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.
[59] The mutant transglutaminase mentioned above, wherein the specific mutation comprises the mutation (A) and the mutation (B).
[60] The mutant transglutaminase mentioned above, wherein the residual activity after heat-treating the mutant transglutaminase at 65°C, pH6.0 for 10 minutes is 15% or more.
[61] The mutant transglutaminase mentioned above, wherein the residual activity after heat-treating the mutant transglutaminase at 75°C, pH6.0 for 10 minutes is 10% or more.
[62] The mutant transglutaminase mentioned above, wherein the residual activity after treating the mutant transglutaminase at pH4.0, 37°C for four hours is 60% or more in term of the relative value to the residual activity after treating the mutant TG at pH6.0, 37°C for four hours which is taken as 100%.
[63] The mutant transglutaminase mentioned above, wherein the wild-type transglutaminase is a protein having an amino acid sequence of a mature transglutaminase of a *Streptomyces* bacterium.
[64] The mutant transglutaminase mentioned above, wherein the *Streptomyces* bacterium is *Streptomyces mobaraensis.*
[65] The mutant transglutaminase mentioned above, wherein the wild-type transglutaminase is any of the following proteins:
   (a) a protein comprising the amino acid sequence of SEQ ID NO: 2;
   (b) a protein comprising the amino acid sequence of SEQ ID NO: 2 but including substitution, deletion, insertion, and/or addition of 1 to 10 amino acid residues; and
   (c) a protein comprising an amino acid sequence having an identity of 90% or higher to the amino acid sequence of SEQ ID NO: 2.
[66] A gene encoding the mutant transglutaminase mentioned above.
[67] A vector comprising the gene mentioned above.
[68] A microorganism having the gene mentioned above.
[69] The microorganism mentioned above, which is a bacterium or a yeast.
[70] The microorganism mentioned above, which is a coryneform bacterium or a bacterium of the family *Enterobacteriaceae.*
[71] The microorganism mentioned above, which is a *Corynebacterium* bacterium or an *Escherichia* bacterium.
[72] The microorganism mentioned above, which is *Corynebacterium glutamicum* or *Escherichia coli.*

### Brief Description of Drawings

[FIG. 1] A diagram showing the compressive stress of processed cheese produced without addition of TG or with addition of wild-type TG or each mutant TG (HT-03, HT-10, or HT-14).
[FIG. 2] A diagram (photograph) showing the shape retention during heating of processed cheese produced without addition of TG or with addition of wild-type TG or mutant TG HT-10.
[FIG. 3] A diagram (photograph) showing the compressive stress and the shape retention during heating of processed cheese produced without addition of TG or with addition of wild-type TG or mutant TG HT-16.
[FIG. 4] A diagram (photograph) showing the shape retention and a result of sensory evaluation of ice cream produced without addition of TG or with addition of mutant TG HT-16.
[FIG. 5] A diagram (photograph) showing the compressive stress and the shape retention during heating of gummy candy produced without addition of TG or with addition of mutant TG HT-16.
[FIG. 6] A diagram showing a result of compression test of jelly (Composition 1) produced with addition of wild-type TG or mutant TG HT-16.
[FIG. 7] A diagram showing a result of compression test of jelly (Composition 2) produced with addition of wild-type TG or mutant TG HT-16.
[FIG. 8] A diagram showing a result of compression test of jelly (Composition 3) produced with addition of wild-type TG or mutant TG HT-16.

### Modes for Carrying out the Invention

### <1> Mutant transglutaminase (mutant TG)

The present invention provides a transglutaminase (TG) having a "specific mutation" as described herein.

The term "transglutaminase (TG)" may refer to a protein having an activity of catalyzing an acyl group transfer reaction between an amide group of a glutamine residue in a protein and a primary amine (EC 2.3.2.13, etc.). This activity is also referred to as "TG activity". A gene encoding TG is also referred to as a "TG gene". Examples of the primary amine include a lysine residue in a protein. That is, the term "TG activity" may specifically refer to an activity of catalyzing a cross-linking reaction between a glutamine residue in a protein and a lysine residue in a protein. The cross-linking reaction may result in formation of an intramolecular cross-link and/or an intermolecular cross-link. The cross-linking reaction may typically result in formation of at least the intermolecular cross-link.

The TG activity can be measured, for example, by the hydroxamate method (Lorand,L., et al.: Anal. Biochem., 44, 221-213(1971)) or by the fluorescence method (Takagi,J., et al.: Anal. Biochem., 153, 296-298(1986)). The term "TG activity" may refer to the TG activity measured by the hydroxamate method, unless otherwise stated.

The procedure for measuring the TG activity by the hydroxamate method is as follows. That is, the TG activity can be determined by incubating the enzyme with the substrate (namely, benzyloxycarbonyl-L-glutaminylglycine and hydroxylamine) at 37°C, pH 6.0 and measuring enzyme- and substrate-dependent formation of hydroxamic acid. The formation of hydroxamic acid can be measured by forming an iron complex of hydroxamic acid in the presence of trichloroacetic acid and measuring an increase in absorbance at 525 nm as an indicator. An amount of enzyme that catalyzes the formation of 1 µmol of hydroxamic acid per minute under the aforementioned conditions is defined as 1 U (unit).

The procedure for measuring the TG activity by the fluorescence method is as follows. That is, the TG activity can be determined by incubating the enzyme with substrates (namely, dimethylated casein and monodansylcadaverine (MDC)) at 37°C, pH 7.5 and measuring enzyme- and substrate-dependent incorporation of MDC into dimethylated casein. The incorporation of MDC can be measured by measuring an increase in fluorescence (excitation wavelength 350 nm, emission wavelength 480 nm) as an indicator.

TG having the "specific mutation" is also referred to as "mutant TG". A gene encoding a mutant TG is also referred to as "mutant TG gene".

TG not having the "specific mutation" is also referred to as "wild-type TG". A gene encoding a wild-type TG is also referred to as "wild-type TG gene". The term "wild-type" referred to herein is used for convenience to distinguish the "wild-type" TG from the "mutant" TG, and the "wild-type" one is not limited to those obtained as natural products, provided that it does not have the "specific mutation". The phrase "TG does not have the "specific mutation"" may mean that TG does not have a mutation selected as the "specific mutation". The wild-type TG may or may not have a mutation not selected as the "specific mutation", provided that it does not have a mutation selected as the "specific mutation".

When a certain wild-type TG and a certain mutant TG are identical to each other except for the presence or absence of the "specific mutation", this certain wild-type TG is also referred to as "wild-type TG corresponding to a certain mutant TG", and this certain mutant TG is also referred to as "mutant TG corresponding to a certain wild-type TG".

Hereafter, the wild-type TG will be explained.

The wild-type TG may or may not have the TG activity, provided that the mutant TG corresponding to the wild-type TG has the TG activity. The wild-type TG may typically have the TG activity.

Examples of the wild-type TG include TGs of Actinomycetes (Appl. Environ. Microbiol., 2003, 69(1), 358-366). Examples of the Actinomycetes include *Streptomyces* bacteria. Examples of the *Streptomyces* bacteria include *Streptomyces mobaraensis, Streptomyces cinnamoneus,* and *Streptomyces griseocarneus.* The term *"Streptomyces* bacteria" also includes bacteria that had been classified to the genus *Streptoverticillium.* For example, the term *"Streptomyces mobaraensis"* also includes bacteria that had been classified to *Streptoverticillium mobaraense* or *Streptoverticillium ladakanum.* In addition, the term *"Streptomyces cinnamoneus"* also includes bacteria that had been classified to *Streptoverticillium cinnamoneum.* In addition, the term *"Streptomyces griseocarneus"* also includes bacteria that had been classified to *Streptoverticillium griseocarneum.* TG, for example, can be expressed in the form of comprising a pro-structure moiety, and then can become a mature protein by removal of the pro-structure moiety. The mature protein of TG is also referred to as "mature TG". That is, specific examples of TGs of the organisms exemplified above include mature TGs of the organisms exemplified above. The nucleotide sequence of a part of the TG gene of *Streptoverticillium mobaraense,* the part encoding the mature TG, is shown as SEQ ID NO: 1, and the amino acid sequence of the mature TG encoded by this gene is shown as SEQ ID NO: 2. That is, the wild-type TG gene may be, for example, a gene having any of the nucleotide sequences of the TG genes of the organisms exemplified above (for example, the nucleotide sequence shown as SEQ ID NO: 1). Also, the wild-type TG may be, for example, a protein having any of the amino acid sequences of the TGs of the organisms exemplified above (for example, the amino acid sequence shown as SEQ ID NO: 2). The expression "a gene or protein has a nucleotide or amino acid sequence" may mean that a gene or protein comprises the nucleotide or amino acid sequence unless otherwise stated, and also include cases where a gene or protein consists of the nucleotide or amino acid sequence.

The wild-type TG gene may also be a variant of any of the wild-type TG genes exemplified above (for example, the gene having the nucleotide sequence shown as SEQ ID NO: 1), provided that the encoded TG does not have the "specific mutation". Similarly, the wild-type TG may be a variant of any of the wild-type TGs exemplified above (for example, the protein having the amino acid sequence shown as SEQ ID NO: 2), provided that the variant does not have the "specific mutation". That is, the term "wild-type TG gene" is not limited to the wild-type TG genes exemplified above (for example, the gene having the nucleotide sequence shown as SEQ ID NO: 1), but may also include variants thereof. Similarly, the term "wild-type TG" is not limited to the wild-type TGs exemplified above (for example, the protein having the amino acid sequence shown as SEQ ID NO: 2), but may also include variants thereof. A gene specified with the type of organism from which the gene is derived is not limited to genes themselves found in that organism, and shall also include genes having any of the nucleotide sequences of the genes found in that organism and variants thereof. A protein specified with the type of organism from which the protein is derived is not limited to proteins themselves found in that organism, and shall also include proteins having any of the amino acid sequences of the proteins found in that organism and variants thereof. That is, for example, the term "TG of Streptoverticillium bacteria" is not limited to TGs themselves found in Streptoverticillium bacteria, and shall also include proteins having any of the amino acid sequences of the TGs found in Streptoverticillium bacteria and variants thereof. Examples of the variants include, for example, homologues and artificially modified versions of the genes and proteins exemplified above.

Homologues of the wild-type TG gene or homologues of the wild-type TG can be easily obtained from public databases by, for example, BLAST search or FASTA search using any of the nucleotide sequences of the wild-type TG genes exemplified above or any of the amino acid sequences of the wild-type TGs exemplified above as a query sequence. Furthermore, homologues of the wild-type TG gene can be obtained by, for example, PCR using a chromosome of various organisms as the template, and oligonucleotides prepared on the basis of any of the nucleotide sequences of the wild-type TG genes exemplified above as primers.

The wild-type TG gene may be a gene encoding a protein having any of the aforementioned amino acid sequences (for example, the amino acid sequence shown as SEQ ID NO: 2) including substitution, deletion, insertion, and/or addition of one or several amino acid residues at one or several positions, provided that the encoded TG does not have the "specific mutation". For example, the encoded protein may have an extended or deleted N-terminus and/or C-terminus. Although the number meant by the term "one or several" used above may differ depending on the positions of amino acid residues in the three-dimensional structure of the protein or the types of amino acid residues, specifically, it may be, for example, 1 to 50, 1 to 40, 1 to 30, 1 to 20, 1 to 10, 1 to 5, or 1 to 3.

The aforementioned substitution, deletion, insertion, or addition of one or several amino acid residues each are a conservative mutation that maintains the original function of the protein. Typical examples of the conservative mutation are conservative substitutions. The conservative substitution is a mutation wherein substitution takes place mutually among Phe, Trp, and Tyr, if the substitution site is an aromatic amino acid; among Leu, Ile, and Val, if it is a hydrophobic amino acid; between Gln and Asn, if it is a polar amino acid; among Lys, Arg, and His, if it is a basic amino acid; between Asp and Glu, if it is an acidic amino acid; and between Ser and Thr, if it is an amino acid having a hydroxyl group. Examples of substitutions considered as conservative substitutions include, specifically, substitution of Ser or Thr for Ala, substitution of Gln, His, or Lys for Arg, substitution of Glu, Gln, Lys, His, or Asp for Asn, substitution of Asn, Glu, or Gln for Asp, substitution of Ser or Ala for Cys, substitution of Asn, Glu, Lys, His, Asp, or Arg for Gln, substitution of Gly, Asn, Gln, Lys, or Asp for Glu, substitution of Pro for Gly, substitution of Asn, Lys, Gln, Arg, or Tyr for His, substitution of Leu, Met, Val, or Phe for Ile, substitution of Ile, Met, Val, or Phe for Leu, substitution of Asn, Glu, Gln, His, or Arg for Lys, substitution of Ile, Leu, Val, or Phe for Met, substitution of Trp, Tyr, Met, Ile, or Leu for Phe, substitution of Thr or Ala for Ser, substitution of Ser or Ala for Thr, substitution of Phe or Tyr for Trp, substitution of His, Phe, or Trp for Tyr, and substitution of Met, Ile, or Leu for Val. Furthermore, such substitution, deletion, insertion, or addition of amino acid residues as mentioned above includes a naturally occurring mutation due to an individual difference, or a difference of species of the organism from which the gene is derived (mutant or variant).

Furthermore, the wild-type TG gene may be a gene encoding a protein having an amino acid sequence showing an identity of, for example, 50% or more, 65% or more, 80% or more, 90% or more, 95% or more, 97% or more, or 99% or more, to the total amino acid sequence of any of the aforementioned amino acid sequences, provided that the encoded TG does not have the "specific mutation".

Furthermore, the wild-type TG gene may be a gene, such as a DNA, that is able to hybridize under stringent conditions with a probe that can be prepared from any of the aforementioned nucleotide sequences (for example, the nucleotide sequence shown as SEQ ID NO: 1), such as a sequence complementary to the whole sequence or a partial sequence of any of the aforementioned nucleotide sequences, provided that the encoded TG does not have the "specific mutation". The term "stringent conditions" may refer to conditions under which a so-called specific hybrid is formed, and a nonspecific hybrid is not formed. Examples of the stringent conditions include those under which highly identical DNAs hybridize to each other, for example, DNAs not less than 50%, 65%, or 80% identical, preferably not less than 90% identical, more preferably not less than 95% identical, still more preferably not less than 97% identical, particularly preferably not less than 99% identical, hybridize to each other, and DNAs less identical than the above do not hybridize to each other, or conditions of washing of typical Southern hybridization, namely, conditions of washing once, preferably 2 or 3 times, at a salt concentration and temperature corresponding to 1 x SSC, 0.1% SDS at 60°C, preferably 0.1 x SSC, 0.1% SDS at 60°C, more preferably 0.1 x SSC, 0.1% SDS at 68°C.

The probe used for the aforementioned hybridization may be a part of a sequence that is complementary to the gene as described above. Such a probe can be prepared by PCR using oligonucleotides prepared on the basis of a known gene sequence as primers and a DNA fragment containing any of the aforementioned genes as a template. As the probe, for example, a DNA fragment having a length of about 300 bp can be used. When a DNA fragment having a length of about 300 bp is used as the probe, in particular, the washing conditions of the hybridization may be, for example, 50°C, 2 x SSC and 0.1% SDS.

Furthermore, since properties concerning degeneracy of codons changes depending on a host, the wild-type TG gene may include substitution of respective equivalent codons for any codons. That is, the wild-type TG gene may be a variant of any of the wild-type TG genes exemplified above due to the degeneracy of the genetic code. For example, the wild-type TG gene may be a gene modified so as to have optimal codons according to codon frequencies in a host to be used.

The term "identity" between amino acid sequences means an identity between the amino acid sequences calculated by blastp with default scoring parameters (namely, Matrix, BLOSUM62; Gap Costs, Existence = 11, Extension = 1; Compositional Adjustments, Conditional compositional score matrix adjustment). The term "identity" between nucleotide sequences means an identity between the nucleotide sequences calculated by blastn with default scoring parameters (namely, Match/Mismatch Scores = 1, -2; Gap Costs = Linear).

Hereafter, the mutant TG will be explained.

The mutant TG has the TG activity. The degree of the TG activity of the mutant TG is not particularly limited so long as the mutant TG can be used for a desired purpose. The TG activity of the mutant TG, for example, may be 50% or more, 60% or more, 70% or more, 80% or more, 90% or more, 100% or more, 120% or more, 150% or more, or 200% or more, may be 10000% or less, 1000% or less, 200% or less, 150% or less, 120% or less, or 100% or less, or may be within a range defined as a non-contradictory combination thereof, of the TG activity of the wild-type TG corresponding to this mutant TG and/or the TG activity of the wild-type TG consisting of the amino acid sequence shown as SEQ ID NO: 2 in terms of the specific activity.

The mutant TG has the "specific mutation" in the amino acid sequence of the wild-type TG.

That is, the mutant TG may be, for example, a protein having the amino acid sequence shown as SEQ ID NO: 2, but having the "specific mutation". The mutant TG may also be, for example, a protein having the amino acid sequence shown as SEQ ID NO: 2, but having the "specific mutation" and further including substitution, deletion, insertion, and/or addition of one or several amino acid residues at position(s) other than the position(s) of the "specific mutation", and having the TG activity.

Also, in other words, the mutant TG may be a protein having the amino acid sequence identical to that of the wild-type TG, except that the mutant TG has the "specific mutation". That is, the mutant TG may be, for example, a protein having the amino acid sequence shown as SEQ ID NO: 2, except that the mutant TG has the "specific mutation". Also, the mutant TG may be, for example, a protein having the amino acid sequence shown as SEQ ID NO: 2, but including substitution, deletion, insertion, and/or addition of one or several amino acid residues, and having the TG activity, except that the mutant TG has the "specific mutation". Also, the mutant TG may be, for example, a protein having an amino acid sequence showing an identity of 80% or more, preferably 90% or more, more preferably 95% or more, still more preferably 97% or more, or particularly preferably 99% or more to the amino acid sequence of SEQ ID NO: 2, and having the TG activity, except that the mutant TG has the "specific mutation".

The mutant TG may comprise another amino acid sequence in addition to such an amino acid sequence of the mutant TG as exemplified above. That is, the mutant TG may be a fusion protein with another amino acid sequence. Furthermore, the mutant TG may be expressed in the form of comprising another amino acid sequence (namely, in the form of a fusion protein with another amino acid sequence), and may eventually lose a part or the whole of the other amino acid sequence. The phrase "a mutant TG comprises another amino acid sequence" means that the mutant TG eventually obtained comprises another amino acid sequence, unless otherwise stated. On the other hand, The phrase "a mutant TG is expressed in the form of comprising another amino acid sequence" means that the mutant TG comprises another amino acid sequence at least at the time of expression thereof, unless otherwise stated, and does not necessarily mean that the mutant TG eventually obtained comprises another amino acid sequence. The same shall apply to the wild-type TG. The "another amino acid sequence" is not particularly limited so long as the mutant TG has the TG activity. The "another amino acid sequence" can be appropriately selected depending on various conditions such as use purpose thereof. Examples of the "another amino acid sequence" include, for example, a peptide tag, a signal peptide (also referred to as "signal sequence"), a pro-structure moiety, and a recognition sequence of a protease. The "another amino acid sequence" may be bound to, for example, either one or both of the N-terminus and C-terminus of the mutant TG. As the "another amino acid sequence", one kind of amino acid sequence may be used, or two or more kinds of amino acid sequences may be used in combination.

Specific examples of the peptide tag include His tag, FLAG tag, GST tag, Myc tag, MBP (maltose binding protein), CBP (cellulose binding protein), TRX (thioredoxin), GFP (green fluorescent protein), HRP (horseradish peroxidase), ALP (alkaline phosphatase), and Fc region of antibody. Examples of the His tag include 6xHis tag. The peptide tag can be utilized for, for example, detection and purification of the expressed mutant TG.

The signal peptide is not particularly limited so long as it functions in a host in which the mutant TG is expressed. Examples of the signal peptide include a signal peptide that is recognized by the Sec system secretory pathway and a signal peptide recognized by the Tat system secretory pathway. Specific examples of the signal peptide that is recognized by the Sec system secretory pathway include signal peptides of cell surface layer proteins of coryneform bacteria. Specific examples of the signal peptides of cell surface layer proteins of coryneform bacteria include the PS1 signal sequence and the PS2 (CspB) signal sequence of *C*. *glutamicum* (Japanese Patent Laid-open (Kohyo) No. 6-502548), and the SlpA (CspA) signal sequence of C. stationis (Japanese Patent Laid-open (Kokai) No. 10-108675). Specific examples of the signal peptide that is recognized by the Tat system secretory pathway include the TorA signal sequence of *E. coli,* the Sufl signal sequence of *E. coli,* the PhoD signal sequence of *Bacillus subtilis,* the LipA signal sequence of *Bacillus subtilis,* and the IMD signal sequence of *Arthrobacter globiformis* (WO2013/118544). The signal peptide can be used for, for example, secretory production of the mutant TG. When secretory production of the mutant TG is carried out by using the signal peptide, the signal peptide may be cleaved at the time of the secretion, and the mutant TG not having the signal peptide may be secreted out of the cell. That is, it is typically acceptable that the mutant TG eventually obtained does not comprises the signal peptide.

Specific examples of the pro-structure moiety include the pro-structure moiety of each of the wild-type TGs exemplified above. The nucleotide sequence of the TG gene of *Streptoverticillium mobaraense* comprising a part encoding the pro-structure moiety is shown as SEQ ID NO: 3, and the amino acid sequence of TG comprising the pro-structure moiety and encoded by this gene is shown as SEQ ID NO: 4. In the SEQ ID NO: 3, positions 1 to 135 correspond to the part encoding the pro-structure moiety, and position 136 and subsequent positions correspond to the part encoding the mature TG (namely, SEQ ID NO: 1). In the SEQ ID NO: 4, positions 1 to 45 correspond to the pro-structure moiety, and position 46 and subsequent positions correspond to the mature TG (namely, SEQ ID NO: 2). The mutant TG, for example, may be expressed in the form of comprising the pro-structure moiety, and then may become a mature protein by removal of the pro-structure moiety. When the mutant TG is expressed in the form of comprising the pro-structure moiety, the mutant TG can be activated by removal of the pro-structure moiety. Hence, it is typically acceptable that the mutant TG eventually obtained does not comprises the pro-structure moiety. Removal of the pro-structure moiety can be carried out by, for example, using a processing enzyme. Examples of the processing enzyme include proteases such as SAM-P45 (Appl. Environ. Microbiol., 2003, 69(1), 358-366) and Alcalase. Furthermore, as described below, when the mutant TG is expressed with a recognition sequence of a protease inserted into the connection part of the pro-structure moiety and the mutant TG, the pro-structure moiety can be removed by using the corresponding protease.

Specific examples of the recognition sequence of a protease include the recognition sequence of the Factor Xa protease and the recognition sequence of the proTEV protease. The recognition sequence of a protease can be used for, for example, cleavage of the expressed mutant TG. Specifically, for example, when the mutant TG is expressed as a fusion protein with the other amino acid sequence such as a peptide tag and a pro-structure moiety, if the recognition sequence of a protease is inserted into the connection part of the mutant TG and the other amino acid sequence, the other amino acid sequence can be cleaved from the expressed mutant TG by using the corresponding protease to obtain the mutant TG not having the other amino acid sequence.

The mutant TG gene is not particularly limited so long as it encodes such a mutant TG as mentioned above. In the present invention, the term "gene" is not limited to DNA, but may include any polynucleotide, so long as it encodes a target protein. That is, the term "mutant TG gene" may refer to any polynucleotide encoding the mutant TG. The mutant TG gene may be DNA, RNA, or a combination thereof. The mutant TG gene may be single-stranded or double-stranded. The mutant TG gene may be a single-stranded DNA or a single-stranded RNA. The mutant TG gene may be a double-stranded DNA, a double-stranded RNA, or a hybrid strand consisting of a DNA strand and an RNA strand. The mutant TG gene may contain both a DNA residue and an RNA residue in a single polynucleotide chain. When the mutant TG gene contains RNA, the aforementioned descriptions concerning DNA, such as those concerning nucleotide sequences exemplified above, may be applied to RNA with appropriately changing wordings to those for RNA as required. The mode of the mutant TG gene can be chosen according to various conditions such as use mode thereof.

Hereafter, the "specific mutation" will be explained.

The "specific mutation" is a mutation of improving thermostability and/or pH stability. That is, the mutant TG has high thermostability and/or high pH stability. The mutant TG may have higher thermostability and/or higher pH stability than that/those of the wild-type TG. The mutant TG may have higher thermostability and/or higher pH stability than, for example, that/those of the wild-type TG corresponding to this mutant TG and/or that/those of the wild-type TG consisting of the amino acid sequence shown as SEQ ID NO: 2. The "specific mutation" may be a mutation of at least improving thermostability.

The term "thermostability" may refer to tolerance against deactivation caused by heat. That is, the phrase "a mutant TG has high thermostability" may mean that the degree of deactivation caused by heat-treating the mutant TG is small, and in other words, may mean that the residual activity after heat-treating the mutant TG is large. Furthermore, the phrase "a mutant TG has higher thermostability than that of a wild-type TG" may mean that the degree of deactivation caused by heat-treating the mutant TG is smaller than the degree of deactivation caused by heat-treating the wild-type TG under the same conditions, and in other words, may mean that the residual activity after heat-treating the mutant TG is larger than the residual activity after heat-treating the wild-type TG under the same conditions.

The phrase "a mutant TG has high thermostability" may mean that, for example, the residual activity after heat-treating the mutant TG is 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, or 90% or more. The phrase "a mutant TG has high thermostability" may also mean that, for example, the residual activity after heat-treating the mutant TG is 1.1 times or more, 1.3 times or more, 1.5 times or more, 2 times or more, 2.5 times or more, 3 times or more, 5 times or more, 10 times or more, 15 times or more, or 20 times or more larger than the residual activity after heat-treating a wild-type TG, such as a wild-type TG corresponding to the mutant TG or a wild-type TG consisting of the amino acid sequence shown as SEQ ID NO: 2, under the same conditions.

Examples of the heat treatment include treatment at 60°C, 65°C, 70°C, 72°C, 75°C, 77°C, or 80°C at pH6.0 for 10 minutes. The phrase "residual activity after heat-treating TG" refers to the ratio of the TG activity after heat-treating TG to the TG activity before heat-treating TG. The phrase "heat-treating TG" refers to putting TG under such conditions of heat treatment as described above.

For example, the residual activity after heat-treating the mutant TG at 60°C, 65°C, 70°C, or 72°C at pH6.0 for 10 minutes may be 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, or 90% or more. Furthermore, for example, the residual activity after heat-treating the mutant TG at 75°C, pH6.0 for 10 minutes may be 5% or more, 10% or more, 15% or more, 20% or more, 25% or more, 30% or more, 35% or more, 40% or more, 45% or more, 50% or more, 55% or more, 60% or more, 65% or more, 70% or more, or 75% or more. Furthermore, for example, the residual activity after heat-treating the mutant TG at 77°C, pH6.0 for 10 minutes may be 5% or more, 10% or more, 15% or more, 20% or more, 25% or more, 30% or more, 35% or more, 40% or more, or 45% or more. Furthermore, for example, the residual activity after heat-treating the mutant TG at 80°C, pH6.0 for 10 minutes may be 5% or more.

The term "pH stability" may refer to tolerance against deactivation under acidic conditions and/or alkaline conditions. Tolerance against deactivation under acidic conditions is also referred to as "acid tolerance". Tolerance against deactivation under alkaline conditions is also referred to as "alkaline tolerance". That is, the phrase "a mutant TG has high pH stability" may mean that the mutant TG has high acid tolerance and/or high alkaline tolerance. The phrase "a mutant TG has high acid tolerance or high alkaline tolerance" may mean that the degree of deactivation caused by treating the mutant TG under acidic conditions or alkaline conditions is small, and in other words, may mean that the residual activity after treating the mutant TG under acidic conditions or alkaline conditions is large. Furthermore, the phrase "a mutant TG has higher acid tolerance or higher alkaline tolerance than that of a wild-type TG" may mean that the degree of deactivation caused by treating the mutant TG under acidic conditions or alkaline conditions is smaller than the degree of deactivation caused by treating the wild-type TG under the same conditions, and in other words, may mean that the residual activity after treating the mutant TG under acidic conditions or alkaline conditions is larger than the residual activity after treating the wild-type TG under the same conditions.

The phrase "a mutant TG has high acid tolerance or high alkaline tolerance" may mean that, for example, the residual activity after treating the mutant TG under acidic conditions or alkaline conditions is 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, or 90% or more. The phrase "a mutant TG has high acid tolerance or high alkaline tolerance" may also mean that, for example, the residual activity after treating the mutant TG under acidic conditions or alkaline conditions is 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, or 90% or more in term of the relative value to the residual activity after treating the mutant TG under control conditions which is taken as 100%. The phrase "a mutant TG has high acid tolerance or high alkaline tolerance" may also mean that, for example, the residual activity after treating the mutant TG under acidic conditions or alkaline conditions is 1.1 times or more, 1.3 times or more, 1.5 times or more, 2 times or more, 2.5 times or more, 3 times or more, 5 times or more, 10 times or more, 15 times or more, or 20 times or more larger than the residual activity after treating a wild-type TG, such as a wild-type TG corresponding to the mutant TG or a wild-type TG consisting of the amino acid sequence shown as SEQ ID NO: 2, under the same conditions.

Examples of the treatment under acidic conditions include treatment at pH5.0, pH4.5, pH4.0, pH3.5, pH3.0, or pH2.5 at 37°C for four hours. Examples of the treatment under alkaline conditions include treatment at pH7.5, pH8.0, pH8.5, pH9.0, pH9.5, or pH10.0 at 37°C for four hours. Examples of the treatment under control conditions include treatment at pH6.0, 37°C for four hours. The phrase "residual activity after treating TG under certain conditions" refers to the ratio of the TG activity after treating TG under the certain conditions to the TG activity before treating TG under the certain conditions. The phrase "treating TG under certain conditions" refers to putting TG under the certain conditions.

For example, the residual activity after treating the mutant TG at pH4.0, 37°C for four hours may be 60% or more, 65% or more, 70% or more, 75% or more, 80% or more, 85% or more, or 90% or more in term of the relative value to the residual activity after treating the mutant TG at pH6.0, 37°C for four hours which is taken as 100%.

Examples of the "specific mutation" include mutations (A) and (B) described below and other known mutations of improving thermostability and/or pH stability. Examples of the known mutations of improving thermostability and/or pH stability include mutations disclosed in WO2010/101256, WO2008/099898, and WO2019094301.

The "specific mutation" may consist of a mutation at one amino acid residue, or may consist of a combination of mutations at two or more amino acid residues. The "specific mutation" may comprise, for example, the mutation (A) and/or the mutation (B). The "specific mutation", for example, may consist of the mutation (A), may consist of the mutation (B), or may consist of a combination of the mutations (A) and (B).

The mutation (A) refers to mutation(s) at the following amino acid residue(s):
D1, Y24, R48, S101, G102, N139, D142, L147, K152, G157, R167, N176, K181, E182, H188, D189, R208, T245, S246, G250, G275, S284, H289, G301, K327.

The mutation (A) may consist of a mutation at one amino acid residue, or may consist of a combination of mutations at two or more amino acid residues. That is, the mutation (A) may comprise, for example, mutation(s) at one or more amino acid residues selected from these amino acid residues. The mutation (A), for example, may consist of a mutation at one amino acid residue selected from these amino acid residues, or may consist of a combination of mutations at two or more amino acid residues selected from these amino acid residues.

Mutation at any amino acid residue may or may not be selected solely.

In one embodiment, particular examples of the mutation (A) include mutations at S101, G157, R208, G250, and G275. That is, the mutation (A) may comprise, for example, mutation(s) at one or more amino acid residues selected from S101, G157, R208, G250, and G275. The mutation (A), for example, may consist of mutation(s) at one or more amino acid residues selected from S101, G157, R208, G250, and G275, or may consist of a combination of mutation(s) at one or more amino acid residues selected from S101, G157, R208, G250, and G275 and mutation(s) at other one or more amino acid residues.

In another embodiment, particular examples of the mutation (A) include mutations at D1, R48, G102, N139, D142, L147, K152, R167, N176, K181, E182, D189, S246, H289, G301, and K327. That is, the mutation (A) may comprise, for example, mutation(s) at one or more amino acid residues selected from D1, R48, G102, N139, D142, L147, K152, R167, N176, K181, E182, D189, S246, H289, G301, and K327. The mutation (A), for example, may consist of mutation(s) at one or more amino acid residues selected from D1, R48, G102, N139, D142, L147, K152, R167, N176, K181, E182, D189, S246, H289, G301, and K327, or may consist of a combination of mutation(s) at one or more amino acid residues selected from D1, R48, G102, N139, D142, L147, K152, R167, N176, K181, E182, D189, S246, H289, G301, and K327 and mutation(s) at other one or more amino acid residues.

In another embodiment, more particular examples of the mutation (A) include mutations at D1, G102, N139, D142, L147, K152, R167, N176, K181, E182, D189, H289, G301, and K327. That is, the mutation (A) may comprise, for example, mutation(s) at one or more amino acid residues selected from D1, G102, N139, D142, L147, K152, R167, N176, K181, E182, D189, H289, G301, and K327. The mutation (A), for example, may consist of mutation(s) at one or more amino acid residues selected from D1, G102, N139, D142, L147, K152, R167, N176, K181, E182, D189, H289, G301, and K327, or may consist of a combination of mutation(s) at one or more amino acid residues selected from D1, G102, N139, D142, L147, K152, R167, N176, K181, E182, D189, H289, G301, and K327 and mutation(s) at other one or more amino acid residues.

In the aforementioned notation used for defining amino acid residues, the numbers represent the positions in the amino acid sequence shown as SEQ ID NO: 2, and the letters at the left side of the numbers represent the amino acid residues at the respective positions in the amino acid sequence shown as SEQ ID NO: 2 (namely, the amino acid residues before modification at the respective positions). That is, for example, "G275" represents G (Gly) residue at position 275 in the amino acid sequence shown as SEQ ID NO: 2.

In any wild-type TG, each of these amino acid residues represents an "amino acid residue corresponding to each of these amino acid residues in the amino acid sequence shown in SEQ ID NO: 2". That is, for example, "G275" in any wild-type TG represents an amino acid residue corresponding to G (Gly) residue at position 275 in the amino acid sequence shown as SEQ ID NO: 2.

Each of the aforementioned mutations may be substitution of an amino acid residue. In each of the aforementioned mutations, the amino acid residue after modification may be any amino acid residue other than the amino acid residue before modification, provided that thermostability and/or pH stability of TG is improved. That is, as the amino acid residue after modification, it is sufficient to select those resulting in improvement in thermostability and/or pH stability of TG. Specific examples of the amino acid residue after modification include amino acid residues selected from K (Lys), R (Arg), H (His), A (Ala), V (Val), L (Leu), I (Ile), G (Gly), S (Ser), T (Thr), P (Pro), F (Phe), W (Trp), Y (Tyr), C (Cys), M (Met), D (Asp), E (Glu), N (Asn), and Q (Gln), provided that the amino acid residue after modification is other than the amino acid residue before modification.

Specific examples of the mutation (A) include the following mutations:
D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), G275A, S284P, H289I, G301W, and K327F.

That is, the mutation (A) may comprise, for example, one or more mutations selected from these mutations. The mutation (A), for example, may consist of one mutation selected from these mutations, or may consist of a combination of two or more mutations selected from these mutations. The mutation (A) may also consist of, for example, a combination of one or more mutations selected from these mutations and other one or more mutations, such as other mutation(s) at one or more amino acid residues selected from D1, Y24, R48, S101, G102, N139, D142, L147, K152, G157, R167, N176, K181, E182, H188, D189, R208, T245, S246, G250, G275, S284, H289, G301, and K327.

Furthermore, in other words, examples of the mutations at D 1, Y24, R48, S101, G102, N139, D142, L147, K152, G157, R167, N176, K181, E182, H188, D189, R208, T245, S246, G250, G275, S284, H289, G301, and K327 include D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), G275A, S284P, H289I, G301W, and K327F, respectively.

Particular examples of the mutation at S101 include S101P.

Particular examples of the mutation at G157 include G157(A, K, R, S). More particular examples of the mutation at G157 include G157(A, R, S). More particular examples of the mutation at G157 include G157(R, S).

Particular examples of the mutation at R208 include R208E.

Particular examples of the mutation at G250 include G250(A, F, S, N, R). More particular examples of the mutation at G250 include G250(N, R, S). More particular examples of the mutation at G250 include G250(R, S).

Particular examples of the mutation at R48 include R48I. For example, when the mutation at R48 is solely selected, the mutation at R48 may be R48I.

Particular examples of the mutation at S246 include S246N. For example, when the mutation at S246 is solely selected, the mutation at S246 may be S246N.

In one embodiment, particular examples of the mutation (A) include S101(G, A, V, I, P, F, N, Q, Y, K, R, E), G157(A, V, I, S, N, K, R, H, D, E), R208(L, A, E), G250(A, V, L, M, P, F, W, S, T, N, Q, Y, K, R, H, D), and G275A. That is, the mutation (A) may comprise, for example, one or more mutations selected from S101(G, A, V, I, P, F, N, Q, Y, K, R, E), G157(A, V, I, S, N, K, R, H, D, E), R208(L, A, E), G250(A, V, L, M, P, F, W, S, T, N, Q, Y, K, R, H, D), and G275A. The mutation (A), for example, may consist of one mutation selected from S101(G, A, V, I, P, F, N, Q, Y, K, R, E), G157(A, V, I, S, N, K, R, H, D, E), R208(L, A, E), G250(A, V, L, M, P, F, W, S, T, N, Q, Y, K, R, H, D), and G275A, or may consist of a combination of two or more mutations selected from S101(G, A, V, I, P, F, N, Q, Y, K, R, E), G157(A, V, I, S, N, K, R, H, D, E), R208(L, A, E), G250(A, V, L, M, P, F, W, S, T, N, Q, Y, K, R, H, D), and G275A. The mutation (A) may also consist of, for example, a combination of one or more mutations selected from S101(G, A, V, I, P, F, N, Q, Y, K, R, E), G157(A, V, I, S, N, K, R, H, D, E), R208(L, A, E), G250(A, V, L, M, P, F, W, S, T, N, Q, Y, K, R, H, D), and G275A and other one or more mutations, such as other mutation(s) at one or more amino acid residues selected from D1, Y24, R48, S101, G102, N139, D142, L147, K152, G157, R167, N176, K181, E182, H188, D189, R208, T245, S246, G250, G275, S284, H289, G301, and K327.

In one embodiment, more particular examples of the mutation (A) include S101P, G157(A, K, R, S), R208E, G250(A, F, S, N, R), and G275A. In one embodiment, more particular examples of the mutation (A) include S101P, G157(A, R, S), R208E, G250(N, R, S), and G275A. In one embodiment, more particular examples of the mutation (A) include S101P, G157(R, S), R208E, G250(R, S), and G275A. That is, the mutation (A) may comprise, for example, one or more mutations selected from S101P, G157(R, S), R208E, G250(R, S), and G275A. The mutation (A), for example, may consist of one mutation selected from S101P, G157(R, S), R208E, G250(R, S), and G275A, or may consist of a combination of two or more mutations selected from S101P, G157(R, S), R208E, G250(R, S), and G275A. The mutation (A) may also consist of, for example, a combination of one or more mutations selected from S101P, G157(R, S), R208E, G250(R, S), and G275A and other one or more mutations, such as other mutation(s) at one or more amino acid residues selected from D1, Y24, R48, S101, G102, N139, D142, L147, K152, G157, R167, N176, K181, E182, H188, D189, R208, T245, S246, G250, G275, S284, H289, G301, and K327.

In the aforementioned notation used for defining mutations, the numbers and the letters at the left side of the numbers represent the same as described above. In the aforementioned notation used for defining mutations, the letters at the right side of the numbers represent the amino acid residues after modification at the respective positions. That is, for example, "G275A" represents a mutation of replacing G (Gly) residue at position 275 in the amino acid sequence shown as SEQ ID NO: 2 with A (Ala) residue. Also, for example, "G157(R, S)" represents a mutation of replacing G (Gly) residue at position 157 in the amino acid sequence shown as SEQ ID NO: 2 with R (Arg) residue or S (Ser) residue.

In any wild-type TG, each of these mutations represents a "mutation corresponding to each of these mutations in the amino acid sequence shown in SEQ ID NO: 2". In any wild-type TG, a "mutation corresponding to a mutation of replacing the amino acid residue at position X in the amino acid sequence shown in SEQ ID NO: 2 with a certain amino acid residue" should be read as a "mutation of replacing an amino acid residue corresponding to the amino acid residue at position X in the amino acid sequence shown in SEQ ID NO: 2 with a certain amino acid residue ". That is, for example, in any wild-type TG, "G275A" represents a mutation of replacing an amino acid residue corresponding to G (Gly) residue at position 275 in the amino acid sequence shown as SEQ ID NO: 2 with A (Ala) residue.

A combination of mutations regarding the mutation (A) is not particularly limited. Examples of the combination of mutations regarding the mutation (A) include the following combinations:
S101P/G157(A, R, S)/G250(N, R, S), S101P/G157(A, R, S)/R208E/G250(N, R, S), and S101P/G157(A, R, S)/R208E/G250(N, R, S)/G275A.

Specific examples of the combination of mutations regarding the mutation (A) include the following combinations:
S101P/G157S/G250R, S101P/G157S/R208E/G250S, S101P/G157A/R208E/G250S, S101P/G157R/R208E/G250S, S101P/G157R/R208E/G250N, S101P/G157R/R208E/G250S/G275A, and S101P/G157S/R208E/G250R/G275A.

Particular examples of the combination of mutations regarding the mutation (A) include S101P/G157S/R208E/G250S, S101P/G157A/R208E/G250S, S101P/G157R/R208E/G250S, S101P/G157R/R208E/G250N, S101P/G157R/R208E/G250S/G275A, and S101P/G157S/R208E/G250R/G275A.

More particular examples of the combination of mutations regarding the mutation (A) include S101P/G157R/R208E/G250S/G275A and S101P/G157S/R208E/G250R/G275A.

That is, the mutation (A) may comprise, for example, any of these combinations. The mutation (A) may consist of, for example, any of these combinations. The mutation (A) may also consist of, for example, a combination of any of these combinations and other one or more mutations, such as other mutation(s) at one or more amino acid residues selected from D1, Y24, R48, S101, G102, N139, D142, L147, K152, G157, R167, N176, K181, E182, H188, D189, R208, T245, S246, G250, G275, S284, H289, G301, and K327.

In the aforementioned notation used for defining combinations, the numbers and the letters at the left and right sides of the numbers represent the same as described above. In the aforementioned notation used for defining combinations, two or more mutations noted together and inserted with "/" represent a double or more multiple mutation. That is, for example, "S101P/G157S/G250R" represents a triple mutation of S101P, G157S, and G250R.

The mutation (B) is a mutation of introducing a disulfide bond.

Examples of the mutation (B) include the following mutations:
D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.

In one embodiment, examples of the mutation (B) include the following mutations:
D3C/G283C, A81C/V311C, E93C/V112C, A106C/D213C, E107C/Y217C, A160C/G228C, S2C/N282C, S2C/G283C, T7C/E58C, P17C/W330C, and D46C/S318C.

Particular examples of the mutation (B) include the following mutations:
D3C/G283C, A81C/V311C, E93C/V112C, A106C/D213C, E107C/Y217C, A160C/G228C, S84C/K121C, R79C/P169C, A113C/P220C, E119C/S299C, and R89C/S116C.

In one embodiment, particular examples of the mutation (B) include the following mutations:
D3C/G283C, A81C/V311C, E93C/V112C, A106C/D213C, E107C/Y217C, and A160C/G228C.

The notation of mutations is the same as the notation of mutations for the mutation (A). For convenience of explanations, a double mutation of introducing one disulfide bond should be regarded as one mutation in explanations of the mutation (B).

The mutation (B) may consist of one mutation, or may consist of a combination of two or more mutations. The mutation (B) may consist of, for example, a combination of two or more, three or more, or four or more mutations. The mutation (B) may specifically consist of, for example, a combination of one, two, three, or four mutations. That is, the mutation (B) may comprise, for example, one or more mutations selected from these mutations. The mutation (B), for example, may consist of one mutation selected from these mutations, or may consist of a combination of two or more mutations selected from these mutations. The mutation (B) may also consist of, for example, a combination of one or more mutations selected from these mutations and other one or more mutations of introducing disulfide bond(s).

A combination of mutations regarding the mutation (B) is not particularly limited.

The mutation (B) may comprise, for example, at least D3C/G283C. That is, examples of the combination of mutations regarding the mutation (B) include combinations of D3C/G283C and other one or more mutations of introducing disulfide bond(s). Specific examples of the combination of mutations regarding the mutation (B) include combinations of D3C/G283C and one or more mutations selected from A81C/V311C, E93C/V112C, A106C/D213C, E107C/Y217C, A160C/G228C, S84C/K121C, R79C/P169C, A113C/P220C, E119C/S299C, and R89C/S116C. In one embodiment, specific examples of the combination of mutations regarding the mutation (B) include combinations of D3C/G283C and one or more mutations selected from A81C/V311C, E93C/V112C, A106C/D213C, E107C/Y217C, and A160C/G228C.

Particular examples of the combination of mutations regarding the mutation (B) include the following combinations:
D3C/G283C/A81C/V311C, D3C/G283C/E93C/V112C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.

In one embodiment, particular examples of combination of mutations regarding the mutation (B) include the following combinations:
D3C/G283C/A81C/V311C, D3C/G283C/E93C/V112C, D3C/G283C/A106C/D213C, D3C/G283C/E107C/Y217C, and D3C/G283C/A160C/G228C.

That is, the mutation (B) may comprise, for example, any of these combinations. The mutation (B) may consist of, for example, any of these combinations. The mutation (B) may also consist of, for example, a combination of any of these combinations and other one or more mutations of introducing disulfide bond(s).

A combination of the mutations (A) and (B) is not particularly limited. Each of the mutations (A) and (B) constituting the combination may consist of one mutation, or may consist of a combination of two or more mutations. As each of the mutations (A) and (B) constituting the combination, for example, any of those exemplified above can be chosen.

Examples of the combination of the mutations (A) and (B) include combinations of the following mutations (A) and (B):
the mutation (A): S101P/G157(A, R, S)/G250(N, R, S), S101P/G157(A, R, S)/R208E/G250(N, R, S), or S101P/G157(A, R, S)/R208E/G250(N, R, S)/G275A;
the mutation (B): one or more mutations selected from D3C/G283C, A81C/V311C, E93C/V112C, A106C/D213C, E107C/Y217C, A160C/G228C, S84C/K121C, R79C/P169C, A113C/P220C, E119C/S299C, and R89C/S116C.

In one embodiment, examples of the combination of the mutations (A) and (B) include combinations of the following mutations (A) and (B):
the mutation (A): S101P/G157(A, R, S)/G250(N, R, S), S101P/G157(A, R, S)/R208E/G250(N, R, S), or S101P/G157(A, R, S)/R208E/G250(N, R, S)/G275A;
the mutation (B): one or more mutations selected from D3C/G283C, A81C/V311C, E93C/V112C, A106C/D213C, E107C/Y217C, and A160C/G228C.

Particular examples of the combination of the mutations (A) and (B) include combinations of the following mutations (A) and (B):
the mutation (A): S101P/G157S/R208E/G250S, S101P/G157A/R208E/G250S, S101P/G157R/R208E/G250S, S101P/G157R/R208E/G250N, S101P/G157R/R208E/G250S/G275A, or S101P/G157S/R208E/G250R/G275A;
the mutation (B): one or more mutations selected from a combination of D3C/G283C and one or more mutations selected from A81C/V311C, E93C/V112C, A106C/D213C, E107C/Y217C, A160C/G228C, S84C/K121C, R79C/P169C, A113C/P220C, E119C/S299C, and R89C/S116C.

In one embodiment, particular examples of the combination of the mutations (A) and (B) include combinations of the following mutations (A) and (B):
the mutation (A): S101P/G157S/R208E/G250S, S101P/G157A/R208E/G250S, S101P/G157R/R208E/G250S, S101P/G157R/R208E/G250N, S101P/G157R/R208E/G250S/G275A, or S101P/G157S/R208E/G250R/G275A;
the mutation (B): a combination of D3C/G283C and one or more mutations selected from A81C/V311C, E93C/V112C, A106C/D213C, E107C/Y217C, and A160C/G228C.

More particular examples of the combination of the mutations (A) and (B) include combinations of the following mutations (A) and (B):
the mutation (A): S101P/G157R/R208E/G250S/G275A or S101P/G157S/R208E/G250R/G275A;
the mutation (B): D3C/G283C, D3C/G283C/A81C/V311C, D3C/G283C/E93C/V112C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, or D3C/G283C/S84C/K121C/A113C/P220C.

In one embodiment, more particular examples of the combination of the mutations (A) and (B) include combinations of the following mutations (A) and (B):
the mutation (A): S101P/G157R/R208E/G250S/G275A or S101P/G157S/R208E/G250R/G275A;
the mutation (B): D3C/G283C, D3C/G283C/A81C/V311C, D3C/G283C/E93C/V112C, D3C/G283C/A106C/D213C, D3C/G283C/E107C/Y217C, or D3C/G283C/A160C/G228C.

That is, the "specific mutation" may comprise, for example, any of these combinations. The "specific mutation" may consist of, for example, any of these combinations. The "specific mutation" may also consist of, for example, a combination of any of these combinations and other one or more mutations, such as other one or more mutations of introducing disulfide bond(s).

The position of amino acid residue referred to in each of the aforementioned mutations is used for convenience for defining the amino acid residue to be modified, and does not necessarily indicate the absolute position in the wild-type TG. That is, the position of amino acid residue in each of the aforementioned mutations represents the relative position based on the amino acid sequence shown in SEQ ID NO: 2, and the absolute position thereof can shift due to deletion, insertion, addition, or the like of amino acid residue(s). For example, if one amino acid residue is deleted or inserted at a position on the N-terminus side of position X in the amino acid sequence shown as SEQ ID NO: 2, the amino acid residue originally at position X is relocated at position X-1 or X+1, but it is still regarded as the "amino acid residue corresponding to the amino acid residue at position X of the amino acid sequence shown as SEQ ID NO: 2". The amino acid residue before modification referred to in each of the aforementioned mutations is used for convenience for defining the amino acid residue to be modified, and is not necessarily conserved in the wild-type TG. That is, when the wild-type TG does not have the amino acid sequence shown as SEQ ID NO: 2, there are cases where the amino acid residue before modification referred to in each of the aforementioned mutations is not conserved. That is, each of the aforementioned mutations may also include a mutation of replacing the amino acid residue before modification referred to in each of the aforementioned mutations, wherein the amino acid residue is not conserved, with another amino acid residue (for example, the amino acid residue after modification referred to in each of the aforementioned mutations). For example, the "mutation at G275" includes not only a mutation of replacing the amino acid residue corresponding to G275, wherein the amino acid residue is conserved (namely, it is G (Gly) residue), with another amino acid residue, but may also include a mutation of replacing the amino acid residue corresponding to G275, wherein the amino acid residue is not conserved (namely, it is not G (Gly) residue), with another amino acid residue. The amino acid residue before modification and the amino acid residue after modification are selected so that they are not identical to each other. For any mutant TG, in particular, the amino acid residue before modification referred to in each of the aforementioned mutations may be conserved in the corresponding wild-type TG.

In the amino acid sequence of a certain TG, which amino acid residue is an "amino acid residue corresponding to the amino acid residue at position X in the amino acid sequence shown in SEQ ID NO: 2" can be determined by aligning the amino acid sequence of the certain TG and the amino acid sequence of SEQ ID NO: 2. The alignment can be carried out by, for example, using known gene analysis software. Examples of specific software include DNASIS produced by Hitachi Solutions, GENETYX produced by Genetyx, and so forth (Elizabeth C. Tyler et al., Computers and Biomedical Research, 24 (1) 72-96, 1991; Barton GJ et al., Journal of Molecular Biology, 198 (2), 327-37, 1987).

### <2> Production of mutant TG

The mutant TG can be produced by, for example, allowing a host having the mutant TG gene to express the gene.

The mutant TG can also be produced by, for example, expressing the mutant TG gene in a cell-free protein synthesis system.

Hereafter, production of the mutant TG using the host having the mutant TG gene is explained in detail.

### <2-1> Host

The host having the mutant TG gene can be obtained by introducing the mutant TG gene into an appropriate host. The phrase "introducing a mutant TG gene into a host" also include modifying a TG gene, such as the wild-type TG gene, possessed by the host so as to encode the mutant TG. The phrase "having a mutant TG gene" is also referred to as "having a mutant TG".

The host is not particularly limited so long as it is able to express a functional mutant TG. Examples of the host include microorganisms, plant cells, insect cells, and animal cells. Particular examples of the host include microorganisms. Examples of the microorganisms include bacteria and yeast. Particular examples of the microorganisms include bacteria.

Examples of the bacteria include bacteria belonging to the family *Enterobacteriaceae,* coryneform bacteria, and *Bacillus* bacteria.

Examples of bacteria belonging to the family *Enterobacteriaceae* include bacteria belonging to the genus *Escherichia, Enterobacter, Pantoea, Klebsiella, Serratia, Erwinia, Photorhabdus, Providencia, Salmonella, Morganella,* or the like. Specifically, bacteria classified into the family *Enterobacteriaceae* according to the taxonomy used in the NCBI (National Center for Biotechnology Information) database (http://www.ncbi.nlm.nih.gov/Taxonomy/Browser/wwwtax.cgi?id=91347) can be used. The *Escherichia* bacteria are not particularly limited, and examples thereof include those classified into the genus *Escherichia* according to the taxonomy known to those skilled in the field of microbiology. Examples of the *Escherichia* bacteria include, for example, those described in the work of Neidhardt et al. (Backmann B.J., 1996, Derivations and Genotypes of some mutant derivatives of Escherichia coli K-12, pp.2460-2488, Table 1, In F.D. Neidhardt (ed.), Escherichia coli and Salmonella Cellular and Molecular Biology/Second Edition, American Society for Microbiology Press, Washington, D.C.). Examples of the *Escherichia* bacteria include, for example, *Escherichia coli.* Specific examples of *Escherichia coli* include, for example, *Escherichia coli* K-12 strains such as W3110 strain (ATCC 27325) and MG1655 strain (ATCC 47076); *Escherichia coli* K5 strain (ATCC 23506); *Escherichia coli* B strains such as BL21 (DE3) strain; and derivative strains thereof. Examples the *Enterobacter* bacterium include, for example, *Enterobacter agglomerans* and *Enterobacter aerogenes.* Examples the *Pantoea* bacteria include, for example, *Pantoea ananatis, Pantoea stewartii, Pantoea agglomerans,* and *Pantoea citrea.* Examples of the *Erwinia* bacteria include *Erwinia amylovora* and *Erwinia carotovora.* Examples of the *Klebsiella* bacteria include *Klebsiellaplanticola.* The bacteria belonging to the family *Enterobacteriaceae* has been recently reclassified into a plurality of families on the basis of comprehensive comparative genome analysis (Adelou M. et al., Genome-based phylogeny and taxonomy of the 'Enterobacteriales': proposal for Enterobacterales ord. nov. divided into the families Enterobacteriaceae, Erwiniaceae fam. nov., Pectobacteriaceae fam. nov., Yersiniaceae fam. nov., Hafniaceae fam. nov., Morganellaceae fam. nov., and Budviciaceae fam. nov., Int. J. Syst. Evol. Microbiol., 2016, 66:5575-5599). However, in the present invention, bacteria previously the family *Enterobacteriaceae* shall be treated as bacteria belonging to the family

### Enterobacteriaceae.

Examples of the coryneform bacteria include bacteria belonging to the genus *Corynebacterium, Brevibacterium, Microbacterium,* or the like.
Specific examples of such coryneform bacteria include the following species.
*Corynebacterium acetoacidophilum*
*Corynebacterium acetoglutamicum*
*Corynebacterium alkanolyticum*
*Corynebacterium callunae*
*Corynebacterium crenatum*
*Corynebacterium glutamicum*
*Corynebacterium lilium*
*Corynebacterium melassecola*
*Corynebacterium thermoaminogenes (Corynebacterium efficiens)*
*Corynebacterium herculis*
*Brevibacterium divaricatum (Corynebacterium glutamicum)*
*Brevibacterium flavum (Corynebacterium glutamicum)*
*Brevibacterium immariophilum*
*Brevibacterium lactofermentum (Corynebacterium glutamicum)*
*Brevibacterium roseum*
*Brevibacterium saccharolyticum*
*Brevibacterium thiogenitalis*
*Corynebacterium ammoniagenes (Corynebacterium stationis)*
*Brevibacterium album*
*Brevibacterium cerinum*
*Microbacterium ammoniaphilum*

Specific examples of the coryneform bacteria include the following strains.
*Corynebacterium acetoacidophilum* ATCC 13870
*Corynebacterium acetoglutamicum* ATCC 15806
*Corynebacterium alkanolyticum* ATCC 21511
*Corynebacterium callunae* ATCC 15991
*Corynebacterium crenatum* AS 1.542
*Corynebacterium glutamicum* ATCC 13020, ATCC 13032, ATCC 13060, ATCC 13869, FERM BP-734
*Corynebacterium lilium* ATCC 15990
*Corynebacterium melassecola* ATCC 17965
*Corynebacterium efficiens* (*Corynebacterium thermoaminogenes*) AJ12340 (FERM BP-1539)
*Corynebacterium herculis* ATCC 13868
*Brevibacterium divaricatum* (*Corynebacterium glutamicum*) ATCC 14020
*Brevibacterium flavum* (*Corynebacterium glutamicum*) ATCC 13826, ATCC 14067, AJ12418 (FERM BP-2205)
*Brevibacterium immariophilum* ATCC 14068
*Brevibacterium lactofermentum* (*Corynebacterium glutamicum*) ATCC 13869
*Brevibacterium roseum* ATCC 13825
*Brevibacterium saccharolyticum* ATCC 14066
*Brevibacterium thiogenitalis* ATCC 19240
*Corynebacterium ammoniagenes* (*Corynebacterium stationis*) ATCC 6871, ATCC 6872
*Brevibacterium album* ATCC 15111
*Brevibacterium cerinum* ATCC 15112
*Microbacterium ammoniaphilum* ATCC 15354

The coryneform bacteria include bacteria that had previously been classified into the genus *Brevibacterium,* but are presently united into the genus *Corynebacterium* (Int. J. Syst. Bacteriol., 41, 255 (1991)). Moreover, *Corynebacterium stationis* includes bacteria that had previously been classified as *Corynebacterium ammoniagenes,* but is presently re-classified into *Corynebacterium stationis* on the basis of nucleotide sequence analysis of 16S rRNA etc. (Int. J. Syst. Evol. Microbiol., 60, 874-879 (2010)).

Examples of the *Bacillus* bacteria include, for example, *Bacillus subtilis, Bacillus amyloliquefaciens, Bacillus pumilus, Bacillus licheniformis, Bacillus megaterium, Bacillus brevis, Bacillus polymixa,* and *Bacillus stearothermophilus.* Specific examples of *Bacillus subtilis* include, for example, the *Bacillus subtilis* 168 Marburg strain (ATCC 6051) and PY79 strain (Plasmid, 1984, 12, 1-9). Specific examples of *Bacillus amyloliquefaciens* include, for example, the *Bacillus amyloliquefaciens* T strain (ATCC 23842) and N strain (ATCC 23845).

Examples of the yeast include yeast belonging to the genus *Saccharomyces* such as *Saccharomyces cerevisiae,* the genus *Candida* such as *Candida utilis,* the genus *Pichia* such as *Pichia pastoris,* the genus *Hansenula* such as *Hansenula polymorpha,* and the genus *Schizosaccharomyces* such as *Schizosaccharomyces pombe.*

These strains are available from, for example, the American Type Culture Collection (Address: 12301 Parklawn Drive, Rockville, Maryland 20852, P.O. Box 1549, Manassas, VA 20108, United States of America). That is, registration numbers are given to the respective strains, and the strains can be ordered by using these registration numbers (refer to http://www.atcc.org/). The registration numbers of the strains are listed in the catalogue of the American Type Culture Collection. These strains can also be obtained from, for example, the depositories at which the strains were deposited.

The mutant TG gene can be obtained by, for example, modifying the wild-type TG gene so that the encoded TG has the "specific mutation". The wild-type TG gene to be modified can be obtained by, for example, cloning from an organism having the wild-type TG gene or chemical synthesis. The mutant TG gene can also be obtained without using the wild-type TG gene. For example, the mutant TG gene may be directly obtained by chemical synthesis. The obtained mutant TG gene may be used as it is, or may be further modified before use. For example, a certain embodiment of the mutant TG gene may be modified to obtain another embodiment of the mutant TG gene.

Genes can be modified by using a known method. For example, an objective mutation can be introduced into a target site of DNA by the site-specific mutagenesis method. That is, for example, the coding region of a gene can be modified by the site-specific mutation method so that a specific site of the encoded protein include substitution, deletion, insertion, and/or addition of amino acid residue(s). Examples of the site-specific mutagenesis method include a method of using PCR (Higuchi, R., 61, in PCR Technology, Erlich, H.A. Eds., Stockton Press, 1989; Carter P., Meth., in Enzymol., 154, 382, 1987), and a method of using a phage (Kramer, W. and Frits, H.J., Meth. in Enzymol., 154, 350, 1987; Kunkel, T.A. et al., Meth. in Enzymol., 154, 367, 1987).

Methods for introducing the mutant TG gene into the host are not particularly limited. It is sufficient that the mutant TG gene is harbored by the host so that the gene is able to be expressed. That is, it is sufficient that the mutant TG gene is harbored by the host so that the gene is able to be expressed under control of a promoter that functions in the host. In the host, the mutant TG gene may be present on a vector autonomously replicable outside a chromosome, such as a plasmid, or may be incorporated into a chromosome. The host may have one copy or two or more copies of the mutant TG gene. The host may have one kind of mutant TG gene, or two or more kinds of mutant TG genes.

The promoter for expressing the mutant TG gene is not particularly limited so long as it functions in the host. The term "promoter that functions in a host" refers to a promoter that shows a promoter activity in the host. The promoter may be a promoter derived from the host, or a heterogenous promoter. The promoter may be the native promoter of the TG gene, or a promoter of another gene. The promoter may be a stronger promoter than the native promoter of the TG gene. Examples of stronger promoters that function in bacteria of the family *Enterobacteriaceae* such as *Escherichia coli* can include, for example, T7 promoter, *trp* promoter, *trc* promoter, *lac* promoter, *tac* promoter, *tet* promoter, *araBAD* promoter, *rpoH* promoter, *msrA* promoter, Pm1 promoter (derived from the genus *Bifidobacterium*)*,* PR promoter, and PL promoter. Examples of stronger promoters that function in coryneform bacteria can include, for example, the artificially modified P54-6 promoter (Appl. Microbiol. Biotechnol., 53, 674-679 (2000)), *pta, aceA, aceB, adh,* and *amyE* promoters inducible in coryneform bacteria with acetic acid, ethanol, pyruvic acid, or the like, and *cspB, SOD,* and *tuf* (EF-Tu) promoters, which are potent promoters capable of providing a large expression amount in coryneform bacteria (Journal of Biotechnology, 104 (2003) 311-323; Appl. Environ. Microbiol., 2005 Dec; 71 (12):8587-96), as well as P2 promoter (WO2018/079684), P3 promoter (WO2018/079684), *lac* promoter, *tac* promoter, *trc* promoter, and F1 promoter (WO2018/179834). Furthermore, as the stronger promoter, a highly-active type of an inherent promoter may also be obtained by using various reporter genes and used. For example, by making the -35 and -10 regions in a promoter region closer to the consensus sequence, the activity of the promoter can be enhanced (WO00/18935). Examples of highly active-type promoter can include various tac-like promoters (Katashkina JI et al., Russian Federation Patent Application No. 2006134574) and pnlp8 promoter (WO2010/027045). Methods for evaluating the strength of promoters and examples of strong promoters are described in the paper of Goldstein et al. (Prokaryotic Promoters in Biotechnology, Biotechnol. Annu. Rev., 1, 105-128 (1995)), and so forth.

Furthermore, a terminator for termination of gene transcription may be located downstream of the mutant TG gene. The terminator is not particularly limited so long as it functions in the host. The terminator may be a terminator derived from the host, or a heterogenous terminator. The terminator may be the native terminator of the TG gene, or a terminator of another gene. Specific examples of the terminator can include, for example, T7 terminator, T4 terminator, fd phage terminator, *tet* terminator, and *trpA* terminator.

The mutant TG gene may be introduced into the host by, for example, using a vector containing the gene. The vector containing the mutant TG gene is also referred to as an expression vector or recombinant vector of the mutant TG gene. The expression vector of the mutant TG gene can be constructed by, for example, ligating a DNA fragment containing the mutant TG gene with a vector that functions in the host. By transforming the host with the expression vector of the mutant TG gene, the host introduced with the vector can be obtained, that is, the gene can be introduced into the host. As the vector, a vector autonomously replicable in the cell of the host can be used. It is preferred that the vector is a multi-copy vector. Furthermore, it is preferred that the vector has a marker such as an antibiotic resistance gene for selection of a transformant. Furthermore, the vector may have a promoter and/or terminator for expressing the introduced gene. The vector may be, for example, a vector derived from a bacterial plasmid, a vector derived from a yeast plasmid, a vector derived from a bacteriophage, cosmid, phagemid, or the like. Specific examples of vector autonomously replicable in *Enterobacteriaceae* bacteria such as *Escherichia coli* can include, for example, pUC19, pUC18, pHSG299, pHSG399, pHSG398, pBR322, pSTV29 (all of these are available from Takara), pACYC184, pMW219 (NIPPON GENE), pTrc99A (Pharmacia), pPROK series vectors (Clontech), pKK233-2 (Clontech), pET series vectors (Novagen), pQE series vectors (QIAGEN), pCold TF DNA (Takara Bio), pACYC series vectors, and the broad host spectrum vector RSF1010. Specific examples of vector autonomously replicable in coryneform bacteria can include, for example, pHM1519 (Agric. Biol. Chem., 48, 2901-2903 (1984)); pAM330 (Agric. Biol. Chem., 48, 2901-2903 (1984)); plasmids obtained by improving these and having a drug resistance gene; plasmid pCRY30 described in Japanese Patent Laid-open (Kokai) No. 3-210184; plasmids pCRY21, pCRY2KE, pCRY2KX, pCRY31, pCRY3KE, and pCRY3KX described in Japanese Patent Laid-open (Kokai) No. 2-72876 and U.S. Patent No. 5,185,262; plasmids pCRY2 and pCRY3 described in Japanese Patent Laid-open (Kokai) No. 1-191686; pAJ655, pAJ611, and pAJ1844 described in Japanese Patent Laid-open (Kokai) No. 58-192900; pCG1 described in Japanese Patent Laid-open (Kokai) No. 57-134500; pCG2 described in Japanese Patent Laid-open (Kokai) No. 58-35197; pCG4 and pCG11 described in Japanese Patent Laid-open (Kokai) No. 57-183799; pPK4 described in U.S. Patent No. 6,090,597; pVK4 described in Japanese Patent Laid-open (Kokai) No. 9-322774; pVK7 described in Japanese Patent Laid-open (Kokai) No. 10-215883; pVK9 described in WO2007/046389; pVS7 described in WO2013/069634; and pVC7 described in Japanese Patent Laid-open (Kokai) No. 9-070291. Specific examples of vector autonomously replicable in coryneform bacteria can also include, for example, variants of pVC7 such as pVC7H2 (WO2018/179834). Upon construction of the expression vector, for example, the mutant TG gene containing a native promoter region may be integrated into a vector as it is, a coding region of the mutant TG gene may be ligated downstream of such a promoter as mentioned above and then may be integrated into a vector, or a coding region of the mutant TG gene may be integrated downstream of a promoter inherently present on a vector.

Vectors, promoters, and terminators available in various microorganisms are disclosed in detail in "Fundamental Microbiology Vol. 8, Genetic Engineering, KYORITSU SHUPPAN CO., LTD, 1987", and those can be used.

The mutant TG gene can also be introduced into the chromosome of the host. The gene can be introduced into the chromosome by, for example, using homologous recombination (Miller, J.H., Experiments in Molecular Genetics, 1972, Cold Spring Harbor Laboratory). Examples of the gene transfer method utilizing homologous recombination can include, for example, a method of using a linear DNA such as Red-driven integration (Datsenko, K.A., and Wanner, B.L., Proc. Natl. Acad. Sci. USA, 97:6640-6645 (2000)), a method of using a plasmid containing a temperature sensitive replication origin, a method of using a plasmid capable of conjugative transfer, a method of using a suicide vector not having a replication origin that functions in a host, and a transduction method using a phage. Only one copy, or two or more copies of a gene may be introduced. For example, by carrying out homologous recombination using a sequence which is present in multiple copies on the chromosome as a target, multiple copies of the gene can be introduced into the chromosome. Examples of such a sequence which is present in multiple copies on the chromosome can include repetitive DNAs, and inverted repeats located at the both ends of a transposon. Alternatively, homologous recombination may be carried out by using an appropriate sequence on the chromosome such as a gene unnecessary for carrying out the present invention as a target. Furthermore, the gene can also be randomly introduced into the chromosome by using a transposon or Mini-Mu (Japanese Patent Laid-open (Kokai) No. 2-109985, U.S. Patent No. 5,882,888, EP 805867 B1). Upon introduction of the gene into the chromosome, for example, the mutant TG gene containing a native promoter region may be integrated into the chromosome as it is, a coding region of the mutant TG gene may be ligated downstream of such a promoter as mentioned above and then may be integrated into the chromosome, or a coding region of the mutant TG gene may be integrated downstream of a promoter inherently present on the chromosome.

Introduction of the gene into the chromosome can be confirmed by, for example, Southern hybridization using a probe having a nucleotide sequence complementary to the whole or a part of the gene, or PCR using primers prepared on the basis of the sequence of the gene.

The transformation method is not particularly limited, and conventionally known methods can be used. Examples of the transformation method include, for example, a method of treating recipient cells with calcium chloride so as to increase the permeability thereof for DNA, which has been reported for the *Escherichia coli* K-12 strain (Mandel, M. and Higa, A., J. Mol. Biol., 1970, 53, 159-162), a method of preparing competent cells from cells which are in the growth phase, followed by transformation with DNA, which has been reported for *Bacillus subtilis* (Duncan, C.H., Wilson, G.A. and Young, F.E., Gene, 1977, 1:153-167), and so forth. Furthermore, as the transformation method, there can also be used a method of making DNA-recipient cells into protoplasts or spheroplasts, which can easily take up recombinant DNA, followed by introducing a recombinant DNA into the DNA-recipient cells, which is known to be applicable to *Bacillus subtilis,* actinomycetes, and yeasts (Chang, S. and Choen, S.N., 1979, Mol. Gen. Genet., 168:111-115; Bibb, M.J., Ward, J.M. and Hopwood, O.A., 1978, Nature, 274:398-400; Hinnen, A., Hicks, J.B. and Fink, G.R., 1978, Proc. Natl. Acad. Sci. USA, 75:1929-1933). Furthermore, as the transformation method, the electric pulse method reported for coryneform bacteria (Japanese Patent Laid-open (Kokai) No. 2-207791) can also be used.

In addition, when the host already has a TG gene, such as the wild-type TG gene, on the chromosome thereof or the like, the TG gene can also be modified to encode the mutant TG, so that the host has the mutant TG gene. The term "introduction of a mutant TG gene" may also include modifying a TG gene possessed by the host to the mutant TG gene. Modification of a TG gene present on the chromosome or the like can be carried out by, for example, natural mutation, mutagenesis treatment, or genetic engineering.

The host may or may not have the wild-type TG gene. In particular, it is acceptable that the host does not have the wild-type TG gene.

The host may have any characteristics so long as the mutant TG can be produced.

### <2-2> Cultivation of host

By culturing the host having the mutant TG gene, the mutant TG can be expressed.

The culture medium to be used is not particularly limited so long as the host can proliferate in it and express the functional mutant TG. As the culture medium, for example, a usual culture medium used for culture of microorganisms such as bacteria and yeast can be used. The culture medium may contain culture medium components such as carbon source, nitrogen source, phosphate source, and sulfur source, as well as other various organic components and inorganic components as required. The types and concentrations of the culture medium components can be appropriately set according to various conditions such as the type of the host.

Specific examples of the carbon source include, for example, saccharides such as glucose, fructose, sucrose, lactose, galactose, xylose, arabinose, blackstrap molasses, hydrolysates of starches, and hydrolysates of biomass; organic acids such as acetic acid, citric acid, succinic acid, and gluconic acid; alcohols such as ethanol, glycerol, and crude glycerol; and fatty acids. As the carbon source, plant-derived materials can be preferably used. Examples of the plant include, for example, corn, rice, wheat, soybean, sugarcane, beet, and cotton. Examples of the plant-derived materials include, for example, organs such as root, stem, trunk, branch, leaf, flower, and seed, plant bodies including them, and decomposition products of these plant organs. The forms of the plant-derived materials at the time of use thereof are not particularly limited, and they can be used in any form such as unprocessed product, juice, ground product, and purified product. Pentoses such as xylose, hexoses such as glucose, or mixtures of them can be obtained from, for example, plant biomass, and used. Specifically, these saccharides can be obtained by subjecting a plant biomass to such a treatment as steam treatment, hydrolysis with concentrated acid, hydrolysis with diluted acid, hydrolysis with an enzyme such as cellulase, and alkaline treatment. Since hemicellulose is generally more easily hydrolyzed compared with cellulose, hemicellulose in a plant biomass may be hydrolyzed beforehand to liberate pentoses, and then cellulose may be hydrolyzed to generate hexoses. Further, xylose may be supplied by conversion from hexoses by, for example, imparting a pathway for converting hexose such as glucose to xylose to the host. As the carbon source, one kind of carbon source may be used, or two or more kinds of carbon sources may be used in combination.

The concentration of the carbon source in the culture medium is not particularly limited so long as the host can proliferate in it and express the functional mutant TG. The concentration of the carbon source in the culture medium may be as high as possible within such a range that production of the mutant TG is not inhibited. Initial concentration of the carbon source in the culture medium may be, for example, usually 5 to 30 w/v%, preferably 10 to 20 w/v%. Further, the carbon source may be additionally supplied to the culture medium as required. For example, the carbon source may be additionally supplied to the culture medium in proportion to decrease or depletion of the carbon source accompanying progress of the cultivation. While the carbon source may be temporarily depleted so long as the mutant TG can be eventually produced, it may be preferable to carry out the culture so that the carbon source is not depleted or the carbon source does not continue to be depleted.

Specific examples of the nitrogen source include, for example, ammonium salts such as ammonium sulfate, ammonium chloride, and ammonium phosphate, organic nitrogen sources such as peptone, yeast extract, meat extract, and soybean protein decomposition products, ammonia, and urea. Ammonia gas and aqueous ammonia used for pH adjustment may also be used as a nitrogen source. As the nitrogen source, one kind of nitrogen source may be used, or two or more kinds of nitrogen sources may be used in combination.

Specific examples of the phosphate source include, for example, phosphate salts such as potassium dihydrogenphosphate and dipotassium hydrogenphosphate, and phosphoric acid polymers such as pyrophosphoric acid. As the phosphate source, one kind of phosphate source may be used, or two or more kinds of phosphate sources may be used in combination.

Specific examples of the sulfur source include, for example, inorganic sulfur compounds such as sulfates, thiosulfates, and sulfites, and sulfur-containing amino acids such as cysteine, cystine, and glutathione. As the sulfur source, one kind of sulfur source may be used, or two or more kinds of sulfur sources may be used in combination.

Specific examples of other various organic and inorganic components include, for example, inorganic salts such as sodium chloride and potassium chloride; trace metals such as iron, manganese, magnesium, and calcium; vitamins such as vitamin B 1, vitamin B2, vitamin B6, nicotinic acid, nicotinamide, and vitamin B 12; amino acids; nucleic acids; and organic components containing these such as peptone, casamino acid, yeast extract, and soybean protein decomposition product. As the other various organic and inorganic components, one kind of component may be used, or two or more kinds of components may be used in combination.

Furthermore, when an auxotrophic mutant strain that requires a nutrient such as amino acids for growth thereof is used, it is preferable to supplement such a required nutrient to the culture medium.

Culture conditions are not particularly limited so long as the host can proliferate in it and express the functional mutant TG. The culture can be carried out with, for example, usual conditions used for culture of microorganisms such as bacteria and yeast. The culture conditions may be appropriately set according to various conditions such as the type of the host. In addition, expression of the mutant TG gene may be induced, as required.

The culture can be carried out by using a liquid culture medium. At the time of the culture, for example, the host cultured on a solid culture medium such as agar medium may be directly inoculated into a liquid culture medium, or the host cultured in a liquid culture medium as seed culture may be inoculated into a liquid culture medium for main culture. That is, the culture may be carried out separately as seed culture and main culture. In such a case, the culture conditions of the seed culture and the main culture may or may not be the same. It is sufficient that the mutant TG is expressed at least during the main culture. The amount of the host contained in the culture medium at the start of the culture is not particularly limited. For example, a seed culture broth showing an OD660 of 4 to 100 may be added to a culture medium for main culture in an amount of 0.1 to 100 mass %, preferably 1 to 50 mass %, at the start of the culture.

The culture can be carried out as batch culture, fed-batch culture, continuous culture, or a combination of these. The culture medium used at the start of the culture is also referred to as "starting medium". The culture medium supplied to the culture system (for example, fermentation tank) in the fed-batch culture or the continuous culture is also referred to as "feed medium". To supply a feed medium to the culture system in the fed-batch culture or the continuous culture is also referred to as "feed". Furthermore, when the culture is carried out separately as seed culture and main culture, the culture schemes of the seed culture and the main culture may or may not be the same. For example, both the seed culture and the main culture may be carried out as batch culture. Alternatively, for example, the seed culture may be carried out as batch culture, and the main culture may be carried out as fed-batch culture or continuous culture.

In the present invention, the various components such as the carbon source may be contained in the starting medium, feed medium, or both. That is, the various components such as the carbon source may be additionally supplied to the culture medium independently or in any combination during the culture. These components each may be supplied once or a plurality of times, or may be continuously supplied. The types of the components contained in the starting medium may or may not be the same as those of the components contained in the feed medium. Furthermore, the concentrations of the components contained in the starting medium may or may not be the same as the concentrations of the components contained in the feed medium. Furthermore, two or more kinds of feed media containing components of different types and/or different concentrations may be used. For example, when feeding is intermittently carried out two or more times, the types and/or concentrations of components contained in the feed medium may or may not be the same for each feeding.

The culture can be carried out, for example, under aerobic conditions. The term "aerobic conditions" may mean that the dissolved oxygen concentration in the culture medium is 0.33 ppm or higher, or preferably 1.5 ppm or higher. The oxygen concentration can be controlled to be, for example, 1 to 50%, preferably about 5%, of the saturated oxygen concentration. The culture can be carried out, for example, with aeration or shaking. The pH of the culture medium may be, for example, pH 3 to 10, preferably pH 4.0 to 9.5. The pH of the culture medium can be adjusted during the culture as required. The pH of the culture medium can be adjusted by using various alkaline and acidic substances such as ammonia gas, aqueous ammonia, sodium carbonate, sodium bicarbonate, potassium carbonate, potassium bicarbonate, magnesium carbonate, sodium hydroxide, potassium hydroxide, calcium hydroxide, and magnesium hydroxide. The culture temperature may be, for example, 20 to 45°C, preferably 25 to 37°C. The culture time may be, for example, 10 to 120 hours. The culture may be continued, for example, until the carbon source contained in the culture medium is consumed, or until the activity of the host is lost.

By culturing the host as described above, a culture broth containing the mutant TG is obtained. The mutant TG may be accumulated in, for example, microbial cells of the host. The term "microbial cell" may be appropriately read as "cell" depending on the type of the host. Furthermore, depending on the type of the host and/or design of the mutant TG gene, for example, the mutant TG may be accumulated in the periplasm, or may be secreted outside the cells.

The mutant TG may be used as it is contained in the culture broth (specifically, the culture medium or cells), or after being purified from the culture broth (specifically, the culture medium or cells). The purification can be carried out to a desired extent. That is, examples of the mutant TG include a purified mutant TG and a fraction containing the mutant TG. In other words, the mutant TG may be used in the form of a purified enzyme, may be used in the form of such a fraction (namely, in the form contained in such a fraction), or may be used in the form of a combination thereof. Such a fraction is not particularly limited so long as it contains the mutant TG in such a manner that the mutant TG can act on the substrate thereof. Examples of such a fraction include a culture broth of the host having the mutant TG gene (namely, the host having the mutant TG), cells collected from the culture broth, a supernatant collected from the culture broth, a processed product thereof (for example, a processed product of cells such as cell disrupt, cell lysate, cell extract, and immobilized cells), and combinations thereof. The term "purified mutant TG" may include a roughly-purified product. Any of these fractions may be independently used, or may be used in combination with a purified mutant TG. The mutant TG may also be used, for example, in the form of not contained in the cells. The mutant TG may also be used, for example, in the form of the composition of the present invention described below.

When the mutant TG is accumulated in the culture medium, for example, a culture supernatant can be obtained by centrifugation or the like, and then the mutant TG can be purified from the culture supernatant. Also, when the mutant TG is accumulated in the cells of the host, for example, the cells can be subject to a treatment such as disruption, lysis, and extraction, and then the mutant TG can be purified from the treated product. The mutant TG can be purified by, for example, known methods used for purification of proteins. Examples of such methods include ammonium sulfate fractionation, ion exchange chromatography, hydrophobic chromatography, affinity chromatography, gel filtration chromatography, and isoelectric precipitation. These methods can be independently used, or can be used in combination as required.

The use purpose of the mutant TG is not particularly limited.

The mutant TG may be used for, for example, modification of a food. The mutant TG may also be used for, for example, production of a food. The food to be produced by using the mutant TG may be a modified food. That is, specifically, the mutant TG may be used for, for example, production of a modified food. In other words, a modified food may be obtained by modification of a food.

Examples of the food include foods that are heated during production. Specific examples of the food include protein-containing foods that are heated during production. Examples of the foods that are heated during production, such as the protein-containing foods that are heated during production, include processed foods such as milk processed foods, egg processed foods, plant processed foods, meat processed foods, seafood processed foods, and gelatin processed foods. The phrases "milk processed food", "egg processed food", "plant processed food", "meat processed food", "seafood processed food", and "gelatin processed food" refers to a food produced by processing milk, a food produced by processing an egg, a food produced by processing a plant, a food produced by processing meat, a food produced by processing seafood, and a food produced by processing gelatin, respectively. In other words, the phrases "milk processed food", "egg processed food", "plant processed food", "meat processed food", "seafood processed food", and "gelatin processed food" may refer to a food produced by at least using milk as a raw material, a food produced by at least using an egg as a raw material, a food produced by at least using a plant as a raw material, a food produced by at least using meat as a raw material, a food produced by at least using seafood as a raw material, and a food produced by at least using gelatin as a raw material, respectively.

Examples of the milk include milks of mammals such as cow milk, goat milk, sheep milk, buffalo milk, reindeer milk, donkey milk, and camel milk. Examples of the egg include eggs of birds such as chicken egg. Examples of the plant include cereals such as beans and wheat variety. Examples of the beans include soybean, pea, sole bean, chickpea, almond, peanut, and Lupin bean. Examples of the wheat variety include wheat, barley, and rye. The phrase "meat" refers to edible meat. Examples of the meat include meats of animals and meats of birds. Examples of the animals include livestock such as cattle, pig, horse, sheep, goat, and rabbit; wild animals such as boar, deer, and bear; marine mammals such as whale, dolphin, and sea lion. Examples of the birds include chicken, turkey, duck, goose, Guinea fowl, quail, and ostrich. Examples of the seafood include fish such as horse mackerel, salmon, cod, puffer fish, kissing fish, sea eel, blue grenadier, and hake; crustaceans such as shrimp and crab; shellfish such as scallop and oyster; and other aquatic products such as squid and octopus. Examples of the gelatin include those obtained from skins or bones of animals such as cattle and pig.

Examples of the milk processed foods include processed cheese, pre-incubated yogurt, ice cream, and margarine. Examples of the egg processed foods include egg omelet, steamed egg custard, and egg tofu. Examples of the plant processed foods include tofu, yuba, noodles, bread, wheat dough, and vegemeat. The phrase "vegemeat" may refer to a food raw material obtained by processing a raw material containing a plant protein into meat-like form or a food produced by using that food raw material. Examples of the plant protein include proteins of the plants exemplified above. Particular examples of the plant protein include proteins of beans. More particular examples of the plant protein include soybean proteins. That is, particular examples of the vegemeat include those produced by using a raw material containing a soybean protein (also referred to as "soybean meat"). Examples of the plant processed foods, meat processed foods, or seafood processed foods include hams, sausages, hamburger steaks, meat dumplings, and paste products. Meat dumplings may also include, for example, those used as a filling of a filling-wrapping dough sheet food such as shaomai, and those used as a filling of a cabbage roll. For example, examples of the sausages or hamburger steaks include sausages or hamburger steaks obtained by processing vegemeat such as soybean meat, sausages or hamburger steaks obtained by processing meat such as beef and pork, and sausages or hamburger steaks obtained by processing seafood such as fish meat. Examples of the paste products include boiled fish paste (kamaboko), imitation crab (kanikamaboko), and deep-fried fish paste (deep-fried kamaboko). Examples of the gelatin processed foods include jellies and gummies.

Examples of the food also include foods having low pH or high pH. Specific examples of the food include protein-containing foods having low pH or high pH. The foods having low pH or high pH, for example, may be those produced by using a raw material having low pH or high pH, or may be those whose production is accompanied by a decrease or increase in pH. pH may be decreased, for example, due to fermentation. That is, examples of the foods whose production is accompanied by a decrease in pH include foods produced by fermentation. The phrase "low pH", for example, may refer to pH5.0 or lower, pH4.5 or lower, pH4.0 or lower, pH3.5 or lower, pH3.0 or lower, or pH2.5 or lower, may refer to pH1.5 or higher, pH2.0 or higher, pH2.5 or higher, pH3.0 or higher, pH3.5 or higher, or pH4.0 or higher, or may refer to a range defined as a non-contradictory combination thereof. The phrase "high pH", for example, may refer to pH7.5 or higher, pH8.0 or higher, pH8.5 or higher, pH9.0 or higher, pH9.5 or higher, or pH10.0 or higher, may refer to pH11.0 or lower, pH10.5 or lower, pH10.0 or lower, pH9.5 or lower, pH9.0 or lower, or pH8.5 or lower, or may refer to a range defined as a non-contradictory combination thereof.

Examples of the foods having low pH include cereals, potatoes, beans, nuts, meat, seafood, gelatin, dairy products, fats and oils, seasonings, beverages, fruit juices, and processed products thereof. Examples of the cereals and processed products thereof include white rice, brown rice, vinegared rice, oatmeal, rice flour, wheat flour, buckwheat flour, bran, and processed products thereof. Examples of the processed products of rice flour include rice cakes and gyuhi. Examples of the processed products of wheat flour include bread, noodles (pasta, etc.), and fu. Examples of the processed products of buckwheat flour include buckwheat noodles. Examples of the potatoes and processed products thereof include white potatoes and processed products thereof. Examples of the beans and processed products thereof include soy milk, chocolate, fried tofu, pea protein, and processed products thereof. Examples of the meat and processed products thereof include such meat as described above and processed products thereof. Examples of the seafood and processed products thereof include shrimp, crab, adductor, herring roe, and processed products thereof. Examples of the dairy products include yogurt, butter, cheese, and ice cream. Examples of the ice cream include those containing yogurt and/or a fruit. Examples of the fats and oils include margarine. Examples of the seasonings include mayonnaise, ketchup, mustard, soy sauce, and miso. Examples of the beverages include coffee-based drinks, nutritional drinks, fruit juice drinks, cocoa, beers, and sake lees. Examples of the processed products of fruit juices include fruit juice gummies. Beverages may be provided as, for example, high viscosity beverages such as jelly beverages and smoothies. Examples of the foods having low pH also include foods having low pH such as foods adjusted to be low pH selected from such foods that are heated during production as exemplified above.

Examples of the foods having high pH include plant beverages such as soy milk, seaweed extracts such as kelp soup stock, and processed products thereof. Examples of the processed products of soy milk include tofu and yuba. Examples of the processed products of seaweed extracts include foods containing seaweed extracts, such as beverages and jelly foods containing seaweed extracts. Examples of the foods having high pH also include foods having high pH such as foods adjusted to be high pH selected from such foods that are heated during production as exemplified above.

Furthermore, a food produced by using the mutant TG may be used as a raw material, to produce another food. For convenience of explanation, a food produced by using the mutant TG is also referred to as "intermediate product", and another food produced by using an intermediate product as a raw material is also referred to as "final product". That is, examples of the food also include such a final product. Examples of the final product include foods containing such a food as exemplified above (for example, processed cheese). Production of a final product by using an intermediate product as a raw material may also result in modification of the final product. The type of modification of the final product may or may not be identical to the type of modification of the intermediate product. The phrase "using a mutant TG for modification or production of a food" may also include cases where the mutant TG is indirectly used for modification or production of a final product via production of the final product by using an intermediate product as a raw material.

Examples of the modification of foods include improvement of physical properties and improvement of flavors. Examples of the improvement of physical properties include an increase in breaking strength, an increase in compressive strength (namely, compressive stress), an increase in shape retention (for example, shape retention during heating and shape retention at an ordinary temperature), prevention of water separation, impartation of a smooth texture, and an increase in viscosity. Particular examples of the improvement of physical properties include an increase in breaking strength, an increase in compressive strength, and an increase in shape retention such as shape retention during heating. Improvement of physical properties can be measured, for example, using equipment for measuring physical properties, such as a texture analyzer. Improvement of physical properties can also be measured, for example, by sensory evaluation by expert panels. For example, improvement of physical properties such as an increase in breaking strength and an increase in compressive strength may be measured as an increase in hardness, suppleness, or elongation by sensory evaluation. Improvement of flavors can be measured, for example, by sensory evaluation by expert panels. Examples of the improvement of flavors include an increase in richness and an increase in fatness. An increase in richness or an increase in fatness may be obtained, for example, in milk processed foods such as ice cream. That is, for example, by using the mutant TG, an effect of improving physical properties and/or an effect of improving flavors may be obtained in a food such as processed cheese and other foods produced in Examples, as compared to cases of not using the mutant TG. Specifically, for example, by producing a food such as processed cheese and other foods produced in Examples using the mutant TG, a food having an improved physical property, such as processed cheese and other foods produced in Examples whose breaking strength is increased, whose compressive strength is increased, whose shape retention such as shape retention during heating is increased, whose water separation is prevented, and/or to which a smooth texture is imparted, may be obtained, as compared to cases of producing the food such as processed cheese and other foods produced in Examples without using the mutant TG.

### <3> Composition of the present invention

The composition of the present invention is a composition containing the mutant TG.

The composition of the present invention may be, for example, a composition to be used for such a use purpose of the mutant TG as exemplified above. That is, the composition of the present invention may be, for example, a composition for modifying a food such as processed cheese. The composition of the present invention may also be, for example, a composition for producing a food such as processed cheese. The composition of the present invention may also be, specifically, for example, a composition for producing a modified food such as modified processed cheese.

The composition of the present invention may consist of the mutant TG, or may contain ingredient(s) other than the mutant TG.

The ingredients other than the mutant TG are not particularly limited so long as the function of the mutant TG is not spoiled. As the ingredients other than the mutant TG, those acceptable depending on the use purpose of the composition of the present invention can be used. Examples of the ingredients other than the mutant TG include ingredients to be blended in foods or pharmaceuticals.

As the composition of the present invention, for example, the mutant TG in such a mode as exemplified above may be used as it is, or may be used after made into a formulation as required. For preparing such a formulation, additive(s) acceptable depending on the use purpose of the composition of the present invention can be used as required. Examples of the additives include excipients, binders, disintegrating agents, lubricants, stabilizers, corrigents, perfumes, diluents, surfactants, and solvents. The additive(s) can be appropriately selected, for example, according to various conditions such as the form of the composition of the present invention.

The form of the composition of the present invention is not particularly limited. The composition of the present invention may be provided, for example, in any form such as the form of powder, flake, tablet, paste, or liquid.

### <4> Method of the present invention

The method of the present invention is a method of using the mutant TG.

In the method of the present invention, the mutant TG may be used for, for example, such a use purpose of the mutant TG as exemplified above. That is, the method of the present invention may be, for example, a method for modifying a food such as processed cheese. The method of the present invention may also be, for example, a method for producing a food such as processed cheese. The method of the present invention may also be, specifically, for example, a method for producing a modified food such as modified processed cheese.

In modification or production of the food, the mutant TG may be used for treating the food raw material. That is, examples of use of the mutant TG include treating the food raw material with the mutant TG. That is, the method of the present invention may be, for example, a method for modifying a food, the method comprising a step of treating the food raw material with the mutant TG. The method of the present invention may also be, for example, a method for producing a food, the method comprising a step of treating the food raw material with the mutant TG. The method of the present invention may also be, specifically, for example, a method for producing a modified food, the method comprising a step of treating the food raw material with the mutant TG. The phrase "treating a food raw material with a mutant TG" is also referred to as "making a mutant TG act on a food raw material". The step of "treating a food raw material with a mutant TG" is also referred to as "treatment step". That is, the method of the present invention may comprise the treatment step.

The food raw material can be appropriately selected according to various conditions such as the type of food. For example, examples of the food raw material when the food is a milk processed food, egg processed food, plant processed food, meat processed food, seafood processed food, or gelatin processed food include milk, egg, plant, meat, seafood, or gelatin, respectively. These food raw material may or may not have been processed. For example, when the food is processed cheese, milk may be used as a food raw material in the form already processed into cheese such as natural cheese. That is, for example, examples of the food raw material when the food is processed cheese include cheese such as natural cheese.

The mutant TG may be used for treating the food raw material in any form that allows the mutant TG to act on the food raw material. The mutant TG may be used for treating the food raw material, for example, in such a form as exemplified above. The mutant TG may be used for treating the food raw material, specifically, for example, in the form of the composition of the present invention. That is, the phrase "treating a food raw material with a mutant TG" also includes cases of treating the food raw material with the composition of the present invention. The mutant TG can be made act on the food raw material by making the mutant TG coexist with the food raw material as it is or after prepared as a desired form such as the form of liquid. For example, the mutant TG may be added to the food raw material, or a treatment solution containing the mutant TG may be mixed with the food raw material. Such operations for making the mutant TG coexist with the food raw material are collectively referred to as "addition" of the mutant TG.

Modification or production of the food may be carried out by using the same food raw material under the same production conditions as a usual food, except that the mutant TG is used. In addition, the food raw material and the production conditions may each be appropriately modified and then used for modification or production of the food. The method of the present invention may comprise a step of producing the food from the food raw material. This step is also referred to as "production step of a food". In addition, the treatment step may also be a step of treating the food raw material with the mutant TG to produce the food.

The mutant TG may be used under heating conditions. For example, when the mutant TG has high thermostability, the mutant TG may be used under heating conditions. Also, for example, when the food is a food that is heated during production, the mutant TG may be used under heating conditions. That is, heating may be carried out during the treatment step. That is, the treatment step may be a step of treating the food raw material with the mutant TG under heating conditions, or may be a step of making the mutant TG act on the food raw material under heating conditions. Also, in other words, the treatment step may be a step of adding the mutant TG to the food raw material and heating the resulting mixture. Carrying out heating at a certain heating temperature during the treatment step is also referred to as "using a mutant TG at a certain temperature".

The mutant TG may be used under acidic conditions or alkaline conditions. For example, when the mutant TG has high acid tolerance or high alkaline tolerance, the mutant TG may be used under acidic conditions or alkaline conditions. Also, for example, when the food is a food having low pH or high pH, the mutant TG may be used under acidic conditions or alkaline conditions. That is, the treatment step may be a step of treating the food raw material with the mutant TG under acidic conditions or alkaline conditions, or may be a step of making the mutant TG act on the food raw material under acidic conditions or alkaline conditions. Carrying out a treatment at a certain pH during the treatment step is also referred to as "using a mutant TG at a certain pH".

The mutant TG may also be used under acidic or alkaline heating conditions.

The mutant TG may be allowed to act on the food raw material at any stage of the production process of the food so long as the mutant TG is used under desired conditions such as the heating conditions, acidic conditions, or alkaline conditions, and a desired effect such as the effect of modifying the food is obtained. That is, the treatment step may be carried out at any stage of the production process of the food so long as a desired effect such as the effect of modifying the food is obtained.

Conditions for carrying out the treatment step such as conditions for carrying out the heating is not particularly limited so long as a desired effect such as the effect of modifying the food is obtained. In other words, it is sufficient that the treatment step such as the heating is carried out so that the mutant TG can sufficiently act on the food raw material. The mutant TG may or may not be deactivated during the treatment step such as the heating so long as the mutant TG can sufficiently act on the food raw material. The conditions for carrying out the treatment step such as the conditions for carrying out the heating can be appropriately set, for example, according to various conditions such as the properties and addition amount of the mutant TG and the type of the food.

When the treatment step is carried out under the heating conditions, as the conditions for carrying out the heating, such as the heating temperature, heating time, heating means, and pH during the heating, for example, conditions for carrying out heating in production of a usual food can be employed as it is, or after appropriate modification. The heating temperature, for example, may be 60°C or higher, 65°C or higher, 70°C or higher, 72°C or higher, or 75°C or higher, may be 100°C or lower, 95°C or lower, 90°C or lower, 85°C or lower, 80°C or lower, 77°C or lower, 75°C or lower, 72°C or lower, or 70°C or lower, or may be within a range defined as a non-contradictory combination thereof. The heating temperature may be, specifically, for example, 60°C to 100°C, 60°C to 90°C, 65°C to 80°C, or 65°C to 75°C. For example, the heating temperature may be 65°C to 80°C or 65°C to 75°C when the food is processed cheese. The heating time, for example, may be 5 minutes or longer, 10 minutes or longer, 15 minutes or longer, 30 minutes or longer, or 1 hour or longer, may be 6 hours or shorter, 3 hours or shorter, 1 hour or shorter, 30 minutes or shorter, 15 minutes or shorter, or 10 minutes or shorter, or may be within a range defined as a non-contradictory combination thereof. The heating time may be, specifically, for example, 5 minutes to 6 hours, 5 minutes to 3 hours, 5 minutes to 1 hour, 5 minutes to 30 minutes, 5 minutes to 15 minutes, 30 minutes to 6 hours, 30 minutes to 3 hours, 30 minutes to 1 hour, 1 hour to 6 hours, or 1 hour to 3 hours. For example, the heating time may be 5 minutes to 30 minutes or 5 minutes to 15 minutes when the food is processed cheese. Examples of the heating means include incubating, baking or roasting, steaming, boiling, and frying. The pH during the heating may be, for example, acidic, neutral, or alkaline. For the pH during the heating, for example, the descriptions concerning the treatment pH when the treatment step is carried out under the acidic conditions or alkaline conditions can also be similarly applied.

When the treatment step is carried out under the acidic conditions or alkaline conditions, as the conditions for carrying out the treatment step, such as the treatment pH, treatment temperature, and treatment time, for example, conditions for a treatment with TG in production of a usual food can be employed as it is, or after appropriate modification. When the treatment step is carried out under the acidic conditions, the treatment pH, for example, may be pH5.0 or lower, pH4.5 or lower, pH4.0 or lower, pH3.5 or lower, pH3.0 or lower, or pH2.5 or lower, may be pH1.5 or higher, pH2.0 or higher, pH2.5 or higher, pH3.0 or higher, pH3.5 or higher, or pH4.0 or higher, or may be within a range defined as a non-contradictory combination thereof. When the treatment step is carried out under the alkaline conditions, the treatment pH, for example, may be pH7.5 or higher, pH8.0 or higher, pH8.5 or higher, pH9.0 or higher, pH9.5 or higher, or pH10.0 or higher, may be pH11.0 or lower, pH10.5 or lower, pH10.0 or lower, pH9.5 or lower, pH9.0 or lower, or pH8.5 or lower, or may be within a range defined as a non-contradictory combination thereof. The treatment temperature, for example, may be 10°C or higher, 20°C or higher, 30°C or higher, 40°C or higher, 50°C or higher, 60°C or higher, 65°C or higher, 70°C or higher, 72°C or higher, or 75°C or higher, may be 100°C or lower, 95°C or lower, 90°C or lower, 85°C or lower, 80°C or lower, 75°C or lower, 72°C or lower, 70°C or lower, 65°C or lower, 60°C or lower, 50°C or lower, 40°C or lower, or 30°C or lower, or may be within a range defined as a non-contradictory combination thereof. For the treatment temperature, for example, the descriptions concerning the heating temperature when the treatment step is carried out under the heating conditions can also be similarly applied. The treatment time, for example, may be 5 minutes or longer, 10 minutes or longer, 15 minutes or longer, 30 minutes or longer, or 1 hour or longer, may be 6 hours or shorter, 3 hours or shorter, 1 hour or shorter, 30 minutes or shorter, 15 minutes or shorter, or 10 minutes or shorter, or may be within a range defined as a non-contradictory combination thereof. The treatment time may be, specifically, for example, 5 minutes to 6 hours, 5 minutes to 3 hours, 5 minutes to 1 hour, 5 minutes to 30 minutes, 5 minutes to 15 minutes, 30 minutes to 6 hours, 30 minutes to 3 hours, 30 minutes to 1 hour, 1 hour to 6 hours, or 1 hour to 3 hours.

The treatment step may be carried out in conjunction with or separately from the production process of the food. Alternatively, a part of the treatment step may be carried out in conjunction with the production process of the food and the remaining part may be carried out separately from the production process of the food.

In the method of the present invention, ingredient(s) other than the ingredients exemplified above (for example, the food raw material and the mutant TG) may also be used so long as a desired effect such as the effect of modifying the food is obtained. Such ingredients are each also referred to as "additional ingredient". Examples of additional ingredients include raw materials that can be used for production of the food and are other than the ingredients exemplified above. The additional ingredients each may be preliminarily mixed with the food raw material, or may be appropriately added to the food raw material during production of the food. For example, when the food objective substance processed cheese, examples of the additional ingredients include emulsifiers and water.

The addition amounts and addition ratios of the ingredients in the method of the present invention are not particularly limited so long as a desired effect such as the effect of modifying the food is obtained. The addition amounts and addition ratios of the ingredients in the method of the present invention can be appropriately set according to various conditions such as the types of the ingredients.

The addition amount of the mutant TG, for example, may be 0.00033 U or more, 0.001 U or more, 0.0033 U or more, 0.01 U or more, 0.033 U or more, 0.1 U or more, 0.33 U or more, or 1 U or more, may be 100 U or less, 33 U or less, 10 U or less, 3.3 U or less, or 1 U or less, or may be within a range defined as a non-contradictory combination thereof, per 1 g of protein contained in the food raw material. The addition amount of the mutant TG may be, specifically, for example, 0.00033 U to 33 U or 0.0033 U to 3.3 U per 1 g of protein contained in the food raw material.

### Examples

Hereinafter, the present invention will be more specifically explained with reference to non-limiting examples.

### Example 1: Construction of mutant transglutaminases (mutant TGs) and analysis of thermostability

In this Example, mutant TGs were constructed and analyzed for thermostability.

### <1> Experimental methods

Quantification of TG and measurement of the TG activity in this Example were carried out according to the following procedure, unless otherwise stated.

### <1-1> Quantification of TG

Quantification of TG was carried out by HPLC analysis using BSA as a standard. Analysis conditions are shown below.
Mobile phase A: 0.1% trifluoroacetic acid (TFA)
Mobile phase B: 0.1% TFA, 80% acetonitrile
Flow rate: 1.0 ml/min
Column temperature: 40°C
Detection: UV 280 nm
Column: Proteonavi, 4.6 × 150 mm, 5 µm (OSAKA SODA CO., LTD.)
Gradient: 0 min (B: 30%), 0-20 min (B: 30-50%), 20-25 min (B: 50-100%), 25-26 min (B: 100%), 26-27 min (B: 100-30%), 27-30 min (B: 30%)

### <1-2> Measurement of TG activity

Measurement of the TG activity was carried out by the hydroxamate method. Solution A (50 mM MES, 100 mM NH₂OH, 10 mM glutathione (reduced form), 30 mM CBZ-Gln-Gly (Z-QG), adjusted to pH 6.0 with NaOH) (500 µL) was added in a 1.5 mL tube, and heated at 37°C for 5 min. After 50 µL of an enzyme solution was added and the reaction was carried out at 37°C for 10 min, 500 µL of solution B (1N HCl, 4% TCA, 1.67% FeCl₃-6H₂O) was added to stop the reaction. A reaction stop solution (200 µL) was added to a 96-well plate, and the absorbance at 525 nm was measured using a plate reader, to obtain an absorbance of an enzyme sample. As a control, the absorbance was measured for a sample similarly obtained by a reaction using 20 mM MES pH 6.0, to obtain an absorbance of a control sample. A difference between the absorbance of the control sample and the absorbance of the enzyme sample was determined. Separately, a calibration curve was prepared using L-glutamic acid-γ-monohydroxamate instead of the enzyme solution, the amount of hydroxamic acid produced was determined from the absorbance difference. An enzymatic activity that produces 1 µmol of hydroxamic acid per minute was defined as 1 U.

### <2> Introduction of mutation into TG

Mutations effective for improving thermostability were comprehensively analyzed in Examples of WO2010/101256, and it was revealed that G157S/A/R, R208A/L/E, and G250R/F/S/N contribute to improvement of thermostability. Hence, in order to confirm synergistic effects of combinations of these mutations, mutations were introduced into a known thermostable mutant TG "MSS2βB" (D3C/G283C/S101P/G157S/G250R; hereinafter referred to as "HT00"; WO2010/101256), to thereby construct mutant TGs HT01 (D3C/G283C/S101P/G157S/R208E/G250S), HT02 (D3C/G283C/S101P/G157A/R208E/G250S), HT03 (D3C/G283C/S101P/G157R/R208E/G250S), and HT04 (D3C/G283C/S101P/G157R/R208E/G250N). The term "HTX" (X is a positive integer) as the name of the mutant TG may also be referred to as "HT-X". The procedure is as follows.

PCR was carried out by using an expression plasmid of the mutant TG HT00 (i.e., an expression plasmid of MSS20B; WO2010/101256) as a template and using PrimeSTAR(R) Max DNA Polymerase (Takara Bio) and primers shown in Table 1, to thereby obtain two kinds of PCR fragments. The two kinds of PCR fragments were mutually ligated by using In-Fusion(R) HD Cloning Kit (Takara Bio), to thereby obtain an expression plasmid of TG introduced with an objective mutation. An expression plasmid of a double mutant was constructed by using the constructed expression plasmid of a single mutant as a template and introducing an additional mutation via the same procedure. An expression plasmid of a triple or more multiple mutant was constructed by using the constructed expression plasmid of a double or more multiple mutant as a template and introducing an additional mutation via the same procedure.

**Table 1 List of primers used for introduction of mutations**

| Mutation | | Fw primer SEQ ID NO | Rv primer SEQ ID NO |
|---|---|---|---|
| G157A | PCR fragment 1 | 7 | 5 |
| | PCR fragment 2 | 6 | 8 |
| G157S | PCR fragment 1 | 9 | 5 |
| | PCR fragment 2 | 6 | 10 |
| G157R | PCR fragment 1 | 11 | 5 |
| | PCR fragment 2 | 6 | 12 |
| R208E | PCR fragment 1 | 13 | 5 |
| | PCR fragment 2 | 6 | 14 |
| G250N | PCR fragment 1 | 15 | 5 |
| | PCR fragment 2 | 6 | 16 |
| G250R | PCR fragment 1 | 17 | 5 |
| | PCR fragment 2 | 6 | 18 |
| G250S | PCR fragment 1 | 19 | 5 |
| | PCR fragment 2 | 6 | 20 |
| G275A | PCR fragment 1 | 21 | 5 |
| | PCR fragment 2 | 6 | 22 |

### <3> Construction of mutant TG-expressing strains

The expression plasmids of mutant TGs constructed in <2> were each introduced into *Corynebacterium glutamicum* YDK010 (WO 2002/081694 A1) by electroporation. YDK010 is a cell surface layer protein PS2 deficient strain of C. *glutamicum* AJ12036 (FERM BP-734). AJ12036 was originally deposited at the Fermentation Research Institute, Agency of Industrial Science and Technology (currently, independent administrative agency, National Institute of Technology and Evaluation, International Patent Organism Depositary, #120, 2-5-8 Kazusakamatari, Kisarazu-shi, Chiba-ken, 292-0818, Japan) on March 26, 1984 as an international deposit, and assigned an accession number of FERM BP-734. Cells after electroporation were cultured on a CM-Dex plate (5 g/L glucose, 10 g/L polypeptone, 10 g/L Yeast extract, 1 g/L KH₂PO₄, 0.4 g/L MgSO₄-7H₂O, 3 g/L urea, 0.01 g/L FeSO₄-7H₂O, 0.01 g/L MnSO₄-5H₂O, 10 µg/L biotin, 1.2 g/L hydrochloric acid hydrolysate of soybeans (as the total nitrogen), adjusted to pH 7.0 with KOH, and 20 g/L Agar) containing 25 mg/L kanamycin at 30°C. An obtained colony was purified on a CM-Dex plate containing 25 mg/L kanamycin and obtained as a mutant TG-expressing strain. The obtained strain was cultured in 3 mL of a CM-Dex liquid medium containing 25 mg/L kanamycin at 30°C for approximately 16 hours, a 0.6 mL-aliquot of the culture broth was mixed with 0.6 mL of 40% glycerol, and stored as a glycerol stock at -80°C.

### <4> Culturing of mutant TG-expressing strains

A small amount of the glycerol stock of the mutant TG-expressing strain obtained in <3> was scraped off, applied on a CM-Dex plate containing 25 mg/L kanamycin, and cultured at 20°C for 3 days. Cells grown on the plate were inoculated in 10 mL of a CM2G medium (5 g/L glucose, 10 g/L polypeptone, 10 g/L Yeast extract, 5 g/L NaCl, 0.2 g/L DL-methionine, adjusted to pH 7.0 with KOH) containing 25 mg/L kanamycin, and cultured using a large test tube at 30°C for 24 hours with shaking. A 2.5 mL-aliquot of the obtained culture broth was inoculated in 50 mL of a TG production medium (60 g/L glucose, 1 g/L MgSO₄-7H₂O, 0.01 g/L FeSO₄-7H₂O, 0.01 g/L MnSO₄-5H₂O, 30 g/L(NH₄)₂SO₄, 1.5 g/L KH₂PO₄, 0.15 g/L DL-methionine, 0.45 mg/L thiamine hydrochloride, 0.45 mg/L Biotin, adjusted to pH 7.5 with KOH) containing 25 mg/L kanamycin and 50 g/L CaCO₃, and cultured using a shake flask at 30°C for 48 hours. For strains expressing TGs introduced with Cys mutations, DTT was added at a final concentration of 3 mM 5 hours after the start of culture. After completion of culture, the culture broth was collected in a plastic bottle, and stored at - 80°C.

### <5> Purification of mutant TGs

The culture broth obtained in <4> was thawed, centrifuged (8000 rpm, 4°C, 20 min), and filtered through a 0.45 µm filter, to obtain a sterilized solution. The sterilized solution was subject to solvent exchange with 20 mM MES buffer (pH 5.5) at room temperature by using Sephadex G25(M) (GE Healthcare). After adjustment to pH 7.0 with NaOH, Alcalase (Sigma-Aldrich, P4860-50ML) was added at 0.5% by weight to the mutant TG, and the reaction was carried out at 30°C for 17 hours, to activate the mutant TG. After completion of the reaction, the resulting solution was adjusted to pH 5.5 with 10% acetic acid, and the whole quantity thereof was applied to a cation exchange column (Resource S 6 mL, GE Healthcare) well equilibrated with 20 mM MES buffer (pH 5.5). After re-equilibration with the same buffer, elution was carried out with a linear concentration gradient of 0 to 0.5 M NaCl, and protein fractions eluted at around 200 mM of NaCl were fractionated on the basis of UV absorption at a wavelength of 280 nm as an indicator. The TG activity and TG amount of each fraction were measured by the method described in Example 1, and fractions near the peak top having almost equal specific activity, excluding fractions having low specific activity, were collected. The collected fractions were subject to solvent exchange with 20 mM phosphate buffer (pH 6.0) by using HiPrep 26/10 Desalting (GE Healthcare) and used as the mutant TG in the following experiments. Both cation exchange chromatography and buffer exchange were carried out at 4°C.

### <6> Evaluation of thermostability of mutant TGs (evaluation of mutants optimized for mutation sites)

The purified enzymes of mutant TGs obtained in <5> were each diluted with 20 mM MES buffer (pH 6.0) to 0.05 mg/mL in terms of BSA, and heat-treated at 65°C or 70°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. As a control of a non-thermostable TG, thermostability of a wild-type TG (prepared from Activa(R)) was analyzed via the same procedure. In addition, as a control of a thermostable TG, thermostability of the mutant TG HT00 (prepared by using the expression plasmid of the mutant TGHT00 (WO2010/101256) according to the procedure of <3> to <5>) was analyzed via the same procedure.

The results are shown in Table 2. The mutant TGs HT01 to HT04 each showed a residual TG activity higher than those of the wild-type TG and the mutant TG HT00. Hence, it was revealed that the thermostability has been improved in the mutant TGs HT01 to HT04.

**Table 2 Residual TG activity after heat treatment (mutants optimized for mutation sites)**

| | No treatment | 65°C | 70°C |
|---|---|---|---|
| HT01 | 100% | 77.9% | 26.1% |
| D3C/G283C/S 101P/G157S/R208E/G250S | | | |
| HT02 | 100% | 77.3% | 27.6% |
| D3C/G283C/S101P/G157A/R208E/G250S | | | |
| HT03 | 100% | 80.0% | 31.0% |
| D3C/G283C/S101P/G157R/R208E/G250S | | | |
| HT04 | 100% | 78.0% | 31.0% |
| D3C/G283C/S101P/G157R/R208E/G250N | | | |
| Wild-type TG | 100% | 3.1% | 3.2% |
| Thermostable TG HT00 | 100% | 76.2% | 15.9% |
| D3C/G283C/S101P/G157S/G250R | | | |

### <7> Evaluation of thermostability of mutant TGs (identification of mutation site that contributes to improvement of thermostability of HT03)

A mutation site that contributes to improvement of thermostability of HT03, which showed the highest thermostability in <6>, was investigated. Expression plasmids of respective mutant TGs designed by introducing single mutations G157R, R208E, and G250S into HT00 were constructed according to the procedure of <2> using the expression plasmid of the mutant TG HT00 (WO2010/101256) as a template and primers shown in Table 1, and purified enzymes of these mutant TGs were prepared according to the procedure of <3> to <5>. The purified enzymes of mutant TGs were each diluted with 20 mM MES buffer (pH 6.0) to 0.05 mg/mL in terms of BSA, and heat-treated at 65°C or 70°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. As a control of a non-thermostable TG, thermostability of a wild-type TG (prepared from Activa(R)) was analyzed via the same procedure. In addition, as controls of thermostable TGs, thermostability of HT00 and HT03 was analyzed via the same procedure.

The results are shown in Table 3. The thermostability of HT00 was improved to a level equivalent to that of HT03 by introduction of R208E, and hence, it was revealed that R208E contributes to improvement of the thermostability of HT03.

**Table 3 Residual TG activity after heat treatment (identification of mutation sites that contribute to improvement of thermostability of HT03**

| | No treatment | 65°C | 70°C |
|---|---|---|---|
| HT00 + G157R | 100% | 1% | 3% |
| HT00 + R208E | 100% | 96% | 55% |
| HT00 + G250S | 100% | 82% | 23% |
| Wild-type TG | 100% | 2% | 3% |
| HT00 | 100% | 90% | 23% |
| HT03 | 100% | 88% | 52% |

### <8> Evaluation of thermostability of mutant TGs (introduction of single mutations into wild-type TG)

Expression plasmids of mutant TGs designed by introducing single mutations shown in Table 4 into the wild-type TG were constructed according to the procedure of <2> using pPSPTG1, which is an expression plasmid of the wild-type TG (Appl. Environ. Microbiol., 2003, 69(1), 358-366), as a template and primers shown in Table 4, and purified enzymes of these mutant TGs were prepared according to the procedure of <3> to <5>. pPSPTG1 is a secretory expression plasmid of TG having a pro-structure moiety of *Streptoverticillium mobaraense,* which is a wild-type TG. The purified enzymes of mutant TGs were each diluted with 20 mM MES buffer (pH 6.0) to 0.05 mg/mL in terms of BSA, and heat-treated at 60°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment was calculated as a relative value to the absorbance difference in the untreated sample which was taken as 100%. This relative value of the absorbance difference was considered as a residual TG activity after the heat treatment. As a control of a non-thermostable TG, thermostability of a wild-type TG (prepared from Activa(R)) was analyzed via the same procedure.

The results are shown in Table 4. Improvement of the thermostability was observed for all the mutant TGs. In particular, it was revealed that G275A contributes to improvement of the thermostability.

**Table 4 Residual TG activity after heat treatment (introduction of mutations into wild-type TG)**

| | 60°C | PCR fragment 1 | | PCR fragment2 | |
|---|---|---|---|---|---|
| | | Fw primer SEQ ID NO | Rv primer SEQ ID NO | Fw primer SEQ ID NO | Rv primer SEQ ID NO |
| Wild-type TG | 15.1% | - | - | - | - |
| D1F | 18.4% | 23 | 5 | 6 | 24 |
| Y24N | 47.9% | 25 | 5 | 6 | 26 |
| Y24G | 35.2% | 27 | 5 | 6 | 28 |
| R48K | 20.8% | 29 | 5 | 6 | 30 |
| R481 | 29.6% | 31 | 5 | 6 | 32 |
| G102N | 20.8% | 33 | 5 | 6 | 34 |
| N139S | 21.8% | 35 | 5 | 6 | 36 |
| D142A | 19.8% | 37 | 5 | 6 | 38 |
| L147W | 19.4% | 39 | 5 | 6 | 40 |
| K152T | 20.6% | 41 | 5 | 6 | 42 |
| G157K | 21.3% | 43 | 5 | 6 | 44 |
| R167G | 25.1% | 45 | 5 | 6 | 46 |
| N176D | 24.1% | 47 | 5 | 6 | 48 |
| K181R | 21.3% | 49 | 5 | 6 | 50 |
| E182D | 20.3% | 51 | 5 | 6 | 52 |
| H188Y | 22.1% | 53 | 5 | 6 | 54 |
| D189I | 19.9% | 55 | 5 | 6 | 56 |
| T245A | 22.0% | 57 | 5 | 6 | 58 |
| S246N | 22.2% | 59 | 5 | 6 | 60 |
| S246R | 26.2% | 61 | 5 | 6 | 62 |
| S246K | 27.8% | 63 | 5 | 6 | 64 |
| G275A | 74.8% | 65 | 5 | 6 | 66 |
| S284P | 36.2% | 67 | 5 | 6 | 68 |
| H289I | 23.5% | 69 | 5 | 6 | 70 |
| G301W | 33.8% | 71 | 5 | 6 | 72 |
| K327F | 34.0% | 73 | 5 | 6 | 74 |

### <9> Evaluation of thermostability of mutant TGs (introduction of G275A mutation into thermostable TGs)

Thermostability of mutant TGs obtained by introducing G275A found in <8> into HT03 and HT00 + R208E, which showed high thermostability in <6> and <7>, was evaluated. Expression plasmids of mutant TGs designed by introducing G275A into HT03 and HT00 + R208E were constructed according to the procedure of <2> using the expression plasmid of the mutant TGs HT03 and HT00 + R208E as a template and primers shown in Table 1, and purified enzymes of these mutant TGs were prepared according to the procedure of <3> to <5>. The purified enzymes of mutant TGs were each diluted with 20 mM MES buffer (pH 6.0) to 0.05 mg/mL in terms of BSA, and heat-treated at 70°C or 72°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. As a control, thermostability of a wild-type TG (prepared from Activa(R)) was analyzed via the same procedure.

The results are shown in Table 5. The thermostability of both HT00 + R208E and HT03 was improved by introduction of G275A, and hence, it was revealed that G275A contributes to improvement of the thermostability. The mutant TG (D3C/G283C/S101P/G157R/R208E/G250S/G275A) obtained by introducing G275A into HT03 was designated as "HT10", and the expression plasmid thereof was designated as "pPSPTG1-HT10". The mutant TG (D3C/G283C/S101P/G157S/R208E/G250R/G275A) obtained by introducing R208E and G275A into HT00 was designated as "HT14", and the expression plasmid thereof was designated as "pPSPTG1-HT14".

**Table 5 Residual TG activity after heat treatment (introduction of G275A)**

| | No treatment | 70°C | 72°C |
|---|---|---|---|
| Wild-type TG | 100% | 4% | 6% |
| HT00 | 100% | 28% | 2% |
| HT00 + R208E | 100% | 54% | 22% |
| HT00 + R208E + G275A | 100% | 95% | 58% |
| (synonym: HT14) | | | |
| HT03 | 100% | 53% | 37% |
| HT03 + G275A (synonym: HT10) | 100% | 95% | 73% |

### <10> Evaluation of thermostability of mutant TGs -introduction of SS bonds into thermostable TGs- (1)

SS bonds were introduced into HT10 (D3C/G283C/S101P/G157R/R208E/G250S/G275A), which showed high thermostability in <9>, and thermostability of the obtained mutant TGs was evaluated.

### <10-1> Introduction of SS bonds into HT10

PCR was carried out by using the TG expression plasmid pPSPTG1-HT10 constructed in <9> as a template and using PrimeSTAR(R) Max DNA Polymerase (Takara Bio) and primers shown in Table 6, to thereby obtain two kinds of PCR fragments. The two kinds of PCR fragments were mutually ligated by using InFusion(R) HD Cloning Kit (Takara Bio), to thereby obtain an expression plasmid of TG introduced with an objective mutation.

**Table 6 List of primers used for introduction of SS bond mutations**

| Mutation | | Fw primer SEQ ID NO | Rv primer SEQ ID NO |
|---|---|---|---|
| A81C/V311C | PCR fragment 1 | 75 | 76 |
| | PCR fragment 2 | 77 | 78 |
| E93C/V112C | PCR fragment 1 | 79 | 80 |
| | PCR fragment 2 | 81 | 82 |
| A106C/D213C | PCR fragment 1 | 83 | 84 |
| | PCR fragment 2 | 85 | 86 |
| E107C/Y217C | PCR fragment 1 | 87 | 88 |
| | PCR fragment 2 | 89 | 90 |
| A160C/G228C | PCR fragment 1 | 91 | 92 |
| | PCR fragment 2 | 93 | 94 |

### <10-2> Evaluation of thermostability

Purified enzymes of mutant TGs were each prepared according to the procedure of <3> to <5>. The obtained purified enzymes of mutant TGs were each diluted with 20 mM MES buffer (pH 6.0) to 0.05 mg/mL in terms of BSA, and heated at 65 to 80°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. Thermostability of a wild-type TG (prepared from Activa(R)), HT00, HT03, and HT10 was analyzed via the same procedure.

The results are shown in Table 7. It was revealed that the thermostability has been significantly improved by introduction of a SS bond. HT10+E93C/V112C (D3C/G283C/S101P/G157R/R208E/G250S/G275A+E93C/V112C) was designated as "HT-16".

**Table 7 Evaluation of thermostability of mutant TGs (introduction of SS bond)**

| | No treatment | 65°C | 70°C | 72°C | 75°C | 77°C | 80°C |
|---|---|---|---|---|---|---|---|
| Wild-type TG | 100% | 3% | 4% | ND | 3% | ND | ND |
| HT00 | 100% | 87% | 24% | ND | 7% | ND | ND |
| HT03 | 100% | 92% | 56% | ND | 5% | ND | ND |
| HT10 | 100% | 103% | 93% | ND | 5% | ND | ND |
| HT10+A81C/V311C | 100% | ND | ND | 77% | 40% | 36% | 6% |
| HT10+E93C/V112C | 100% | ND | ND | 91% | 76% | 47% | 7% |
| HT10+A106C/D213C | 100% | ND | ND | 90% | 71% | 31% | 9% |
| HT10+E107C/Y217C | 100% | ND | ND | 89% | 69% | 34% | 6% |
| HT10+A160C/G228C | 100% | ND | ND | 90% | 70% | 47% | 5% |

| | | | | | | | |
|---|---|---|---|---|---|---|---|
| ND: No data | | | | | | | |

### <11> Evaluation of thermostability of mutant TGs -introduction of SS bonds into thermostable TGs- (2)

SS bonds were introduced into HT10 (D3C/G283C/S101P/G157R/R208E/G250S/G275A), which showed high thermostability in <9>, and thermostability of the obtained mutant TGs was evaluated.

### <11-1> Introduction of SS bonds into HT10

Two times of a site-direct mutagenesis method by PCR using the TG expression plasmid pPSPTG1-HT10 constructed in <9> as a template and using PrimeSTAR(R) Max DNA Polymerase (Takara Bio) and primers shown in Table 8 was carried out sequentially, to thereby obtain expression plasmids of TGs introduced with objective mutations.

**Table 8 List of primers used for introduction of SS bond mutations**

| Mutation | | Fw primer SEQ ID NO | Rv primer SEQ ID NO |
|---|---|---|---|
| S84C/K121C | S84C | 95 | 96 |
| | K121C | 97 | 98 |
| R79C/P169C | P79C | 99 | 100 |
| | P169C | 101 | 102 |
| A113C/P220C | A113C | 103 | 104 |
| | P220C | 105 | 106 |
| E119C/S299C | E119C | 107 | 108 |
| | S229C | 109 | 110 |
| R89C/S116C | R89C | 111 | 112 |
| | S116C | 113 | 114 |

### <11-2> Culturing of mutant TG-expressing strains

A glycerol stock of a mutant TG-expressing strain for each of the mutant TGs constructed in <11-1> was obtained according to the procedure of <3>. A small amount of the obtained glycerol stock of the mutant TG-expressing strain was scraped off, applied on a CM-Dex plate containing 25 mg/L kanamycin, and cultured at 30°C for 2 days. Cells grown on the plate were inoculated in 800 µL of a CM2G medium (5 g/L glucose, 10 g/L polypeptone, 10 g/L Yeast extract, 5 g/L NaCl, 0.2 g/L DL-methionine, adjusted to pH 7.0 with KOH) containing 25 mg/L kanamycin, and cultured using a 96 well plate at 30°C for 24 hours with shaking. A 40 µL-aliquot of the obtained culture broth was inoculated in 760 µL of a TG production medium (60 g/L glucose, 1 g/L MgSO₄-7H₂O, 0.01 g/L FeSO₄-7H₂O, 0.01 g/L MnSO₄-5H₂O, 30 g/L(NH₄)₂SO₄, 1.5 g/L KH₂PO₄, 0.15 g/L DL-methionine, 0.45 mg/L thiamine hydrochloride, 0.45 mg/L Biotin, adjusted to pH 7.5 with KOH) containing 25 mg/L kanamycin and 50 g/L CaCO₃, and cultured using a 96 well plate at 30°C for 48 hours. For strains expressing TGs introduced with Cys mutations, DTT was added at a final concentration of 3 mM 5 hours after the start of culture. After completion of culture, the culture broth was stored at -80°C.

In addition, a culture broth of a mutant TG-expressing strain for each of the mutant TGs constructed in <10-1> was obtained and stored at -80°C according to the procedure of <3> and <4>.

### <11-3> Preparation of mutant TG solutions

The culture broth obtained in <11-2> was thawed, centrifuged (5000 rpm, 20°C, 10 min), to obtain a culture supernatant. The protein concentration of the culture supernatant was quantified, and the culture supernatant was diluted so as to obtain a protein concentration of 0.2 mg/mL. The protein concentration was quantified by the Bradford method using a kit of ThermoFisher Scientific with BSA as a standard. A 1000 µL-aliquot of the diluted solution was added with 10 µL of 0.4% Alcalase (Sigma-Aldrich, P4860-50ML), and the reaction was carried out at 30°C for 17 hours, to activate the mutant TG. After completion of the reaction, the resulting solution was added with 10 µL of 100 mM PFSF and left to stand at a room temperature for 1 hour, to thereby obtain a mutant TG solution.

### <11-4> Evaluation of thermostability

The obtained mutant TG solutions were each diluted with 20 mM MES buffer (pH 6.0) to 0.05 mg/mL in terms of BSA, and heated at 65 to 80°C for 10 minutes with a thermal cycler. An enzymatic reaction was carried out using 50 µL of the sample after the heat treatment according to the procedure of <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. Thermostability of a wild-type TG (prepared from Activa(R)), HT00, and HT10 was analyzed via the same procedure.

The results are shown in Table 9. It was revealed that the thermostability has been significantly improved by introduction of SS bond(s).

**Table 9 Evaluation of thermostability of mutant TGs (introduction of SS bond)**

| | No treatment | 70°C | 75°C | 77°C | 80°C |
|---|---|---|---|---|---|
| Wild-type TG | 100% | ND | ND | ND | ND |
| HT10 | 100% | 69.8 | 10.7 | 12.6 | 9.6 |
| HT10+A81C/V311C | 100% | 72.6 | 29.2 | 21.4 | 8.2 |
| HT10+E93C/V112C | 100% | 98.1 | 63.1 | 26.0 | 8.4 |
| HT10+A106C/D213C | 100% | 95.2 | 46.4 | 9.0 | 4.0 |
| HT10+E107C/Y217C | 100% | 106.3 | 48.2 | 11.5 | 5.7 |
| HT10+A160C/G228C | 100% | 85.9 | 51.8 | 19.0 | 9.7 |
| HT10+S84C/K121C | 100% | 83.3 | 55.5 | 33.4 | 19.7 |
| HT10+R79C/P169C | 100% | 68.8 | 17.7 | 15.1 | 8.7 |
| HT10+A113C/P220C | 100% | 43.9 | 7.6 | 4.6 | 2.3 |
| HT10+E119C/S299C | 100% | 85.0 | 33.7 | 30.5 | 17.9 |
| HT10+R89C/S116C | 100% | 83.8 | 32.6 | 24.7 | 15.1 |
| HT10+E93C/V112C+E107C/Y217C | 100% | 100.5 | 83.8 | 67.4 | 16.8 |
| HT10+E93C/V112C+A113C/P220C | 100% | 88.6 | 68.1 | 43.9 | 12.5 |
| HT10+E93C/V112C+E119C/S299C | 100% | 99.8 | 64.0 | 32.4 | 11.7 |
| HT10+E107C/Y217C+S84C/K121C | 100% | 82.4 | 66.6 | 52.9 | 25.9 |
| HT10+S84C/K121C+A113C/P220C | 100% | 84.6 | 66.4 | 48.4 | 19.4 |

### Example 2: Preparation and analysis of processed cheese using mutant transglutaminases (mutant TGs)

In this example, an effect of mutant TGs on physical properties of processed cheese was evaluated.

### <1> Evaluation of mutant TGs HT-03, HT-10, and HT-14

### <1-1> Preparation of processed cheese

Processed cheese was prepared using RVA apparatus (Rapid Visco Analyzer, Perten Instruments). Red cheddar cheese (Ito-Yokado, U.S.A.), trisodium polyphosphate (Taihei Chemical Industrial Co., Ltd.), and city water were weighed to 21 g, 0.6 g, and 8.4 g, respectively, in an aluminum container for RVA. A wild-type TG (prepared from a strain expressing pPSPTG1) or each mutant TG (HT-03, HT-10, or HT-14; obtained in Example 1) was preliminarily prepared as an aqueous solution, and added so that the TG activity per 1 g of protein in cheese was 2 U. For each sample to which the enzyme solution was added, the amount of city water added and mixed was reduced by the amount of the enzyme solution added. After addition of the enzyme, the aluminum vessel was quickly set in the RVA apparatus and heated and stirred at 70°C for 11 minutes. The stirring intensity was set at 200 rpm. Before the stirring step, a step of raising the temperature to 70°C for 30 seconds without stirring was carried out. After completion of the heating and stirring, the temperature was raised to 85°C for 1 minute, followed by heating and stirring for 5 minutes, to thereby deactivate the enzyme. The aluminum container was then removed and the contents were poured in approximately 10 g portions into approximately 30-mL plastic cups, and stored at 5°C overnight for cooling.

### <1-2> Measurement of compressive stress values

It is known that the reaction of TG forms covalent bonds between lysine or glutamine residues, which increases the compressive stress of protein-containing foods. Hence, the compressive stress of the prepared processed cheese was measured with a texture analyzer as an indicator of the reactivity of each TG. The measurement conditions are shown below. Two subdivided samples were used per sample, to carry out the analysis with n=2.

### <Measurement conditions with texture analyzer>

Plunger used: Cylinder type (1 cm in diameter)
Contact Force: 0.5 g
Test Mode: Compression
Test speed: 0.5 mm/sec
Target mode: Strain
Strain: 90%
Trigger type: Auto
Trigger force: 0.5 g

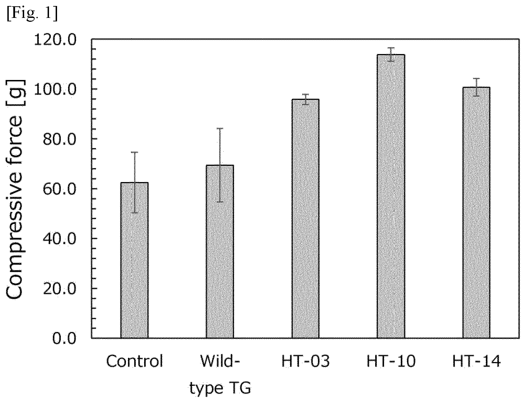

The results are shown in Figure 1. No significant difference in the compressive stress value was observed between the wild-type TG-added product and the TG-free product (Control), and the wild-type TG did not contribute to improvement of the compressive stress value. On the other hand, all the mutant TGs contributed to improvement of the compressive stress value. In particular, HT-10 showed the highest effect of improving the compressive stress, and the compressive stress value of the HT-10-added product was approximately twice that of the TG-free product (Control).

### <1-3> Measurement of shape retention during heating

It is known that improving the shape retention of processed cheese during heating is an object and an added value when the processed cheese is used in processed products for which a heating step is carried out. The shape retention during heating of the HT-10-added product, which showed the highest compressive stress value in <2>, with that of the wild-type TG-added product and the TG-free product. Each processed cheese was heated at 150°C for 1 minute using an electronic oven (SHARP, Healsio), and the melting behavior was observed.

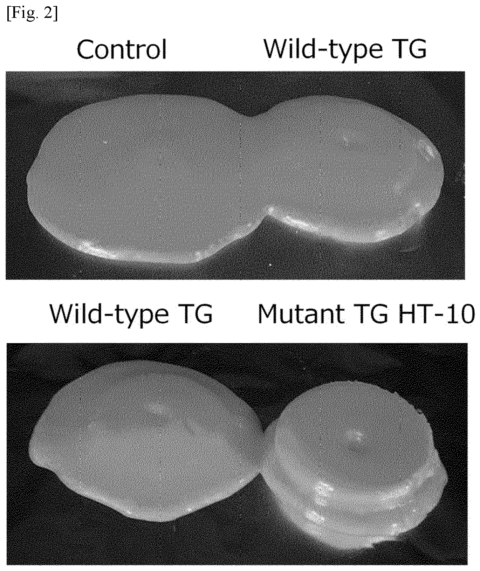

The results (photographs after 1 minute of heating) are shown in Figure 2. The wild-type TG-added product and the TG-free product were almost completely melted, but the wild-type TG-added product showed shape retention slightly higher than that of the TG-free product. In addition, the HT-10-added product mostly retained its shape before heating, whereas the wild-type TG-added product and the TG-free product were almost completely melted. Hence, it was revealed that HT-10 contributes to improvement of the shape retention during heating.

### <2> Evaluation of mutant TG HT-16

### <2-1> Preparation of processed cheese

Processed cheese was prepared according to the procedure of <1-1> using the wild-type TG (prepared from a strain expressing pPSPTG1) or the mutant TG HT-16 (obtained in Example 1) as TG.

### <2-2> Measurement of compressive stress values

The compressive stress of the prepared processed cheese was measured with a texture analyzer. The measurement conditions are shown below. Two subdivided samples were used per sample, to carry out the analysis with n=2.

### <Measurement conditions with texture analyzer>

Plunger used: Cylinder type (12.7 mm in diameter)
Contact Force: 0.5 g
Test Mode: Compression
Test speed: 1.0 mm/sec
Target mode: Distance
Distance: 5 mm
Trigger type: Auto
Trigger force: 0.5 g

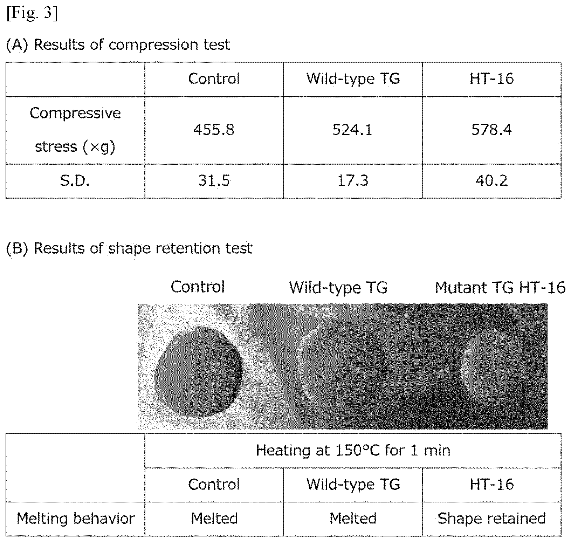

The results are shown in Figure 3, panel (A). It was observed that the addition of TGs provided an effect of improving the compressive stress. In addition, it was observed that the addition of the mutant TG HT-16 provided a higher effect of improving the compressive stress than that provided by the addition of the wild-type TG.

### <2-3> Measurement of shape retention during heating

The shape retention during heating of the prepared processed cheese was measured according to the procedure of <1-3>.

The results (photographs after 1 minute of heating) are shown in Figure 3, panel (B). The wild-type TG-added product and the TG-free product were almost completely melted. On the other hand, the HT-16-added product mostly retained its shape before heating. Hence, it was revealed that HT-16 contributes to improvement of the shape retention during heating.

### Example 3: Analysis of acid tolerance of mutant transglutaminases (mutant TGs)

In this Example, acid tolerance of mutant TGs were analyzed.

The purified enzymes of the mutant TGs shown in Table 10 (obtained in Example 1) were each diluted with 20 mM sodium acetate buffer (pH 4.0 or 5.0) or 20 mM MES buffer (pH 6.0) to 0.56 U/mL, and treated at 37°C for four hours. The residual TG activity after treatment at each pH was measured by the hydroxamate method. Solution A (50 mM MES, 100 mM NH₂OH, 10 mM glutathione (reduced form), 30 mM CBZ-Gln-Gly (Z-QG), adjusted to pH 6.0 with NaOH) (500 µL) was added in a 1.5 mL tube, and heated at 37°C for 5 min. After 150 µL of an enzyme solution after treatment at each pH was added and the reaction was carried out at 37°C for 30 min, 500 µL of solution B (1N HCl, 4% TCA, 1.67% FeCl₃-6H₂O) was added to stop the reaction. A reaction stop solution (200 µL) was added to a 96-well plate, and the absorbance at 525 nm was measured using a plate reader, to obtain an absorbance of an enzyme sample. As a control, the absorbance was measured for a resulting sample similarly obtained by a reaction using 20 mM MES pH 6.0, to obtain an absorbance of a control sample. A difference between the absorbance of the control sample and the absorbance of the enzyme sample was determined. The absorbance difference in the sample after treatment at each pH was calculated as a relative value to the absorbance difference in the sample after treatment at pH 6.0 which was taken as 100%. This relative value of the absorbance difference was considered as a relative value of the residual TG activity after treatment at each pH to the residual TG activity after treatment at pH 6.0 which was taken as 100%. The acid tolerance of a wild-type TG (prepared from Activa(R)) was analyzed via the same procedure.

The results are shown in Table 10. No decrease in the residual TG activity due to acid treatment was observed for the mutant TGs, whereas a decrease in the residual TG activity due to acid treatment was observed for the wild-type TG. Hence, it was revealed that the acid tolerance has been improved in the mutant TGs.

**Table 10 Relative values of residual TG activity after treatment at respective pH**

| | pH 4.0 | pH 5.0 | pH 6.0 |
|---|---|---|---|
| Wild-type TG | 55% | 93% | 100% |
| HT00 | 115% | 107% | 100% |
| HT03 | 112% | 102% | 100% |
| HT10 | 110% | 104% | 100% |
| HT10+E93C/V112C | 104% | 101% | 100% |
| HT10+A160C/G228C | 111% | 103% | 100% |

### Example 4: Construction of mutant transglutaminases (mutant TGs) and analysis of thermostability

### <1> Introduction of SS bonds into wild-type TG and mutant TG (D3C/G283C)

Mutant TG expression plasmids designed by introducing mutations into the TG gene of the TG expression plasmid pPSPTG1 were prepared by total synthesis. The mutant names and mutation sites are listed in Table 11.

**Table 11**

| Mutant name | Mutation site |
|---|---|
| WT | - |
| WT1 | A81C/C311C |
| WT2 | E93C/V112C |
| WT3 | A106C/D213C |
| WT4 | E107C/Y217C |
| WT5 | A160C/G228C |
| SS | D3C/G283C |
| SS1 | D3C/G283C/A81C/C311C |
| SS2 | D3C/G283C/E93CN112C |
| SS3 | D3C/G283C/A106C/D213C |
| SS4 | D3C/G283C/E107C/Y217C |
| SS5 | D3C/G283C/A160C/G228C |

### <2> Evaluation of thermostability of mutant TGs introduced with SS bonds

The thermostability of the obtained mutant TGs was evaluated. Based on the results of WO2008/099898, it is expected that WT1 to WT5 show thermostability equivalent to or less than that of the wild-type TG (WT). On the other hands, based on the results of Example 1, it is expected that SS1 to SS5 show improved thermostability compared to SS. Purified enzymes of mutant TGs were each prepared according to the procedure of Example 1 <3> to <5>. The obtained purified enzymes of mutant TGs were each diluted with 20 mM MES buffer (pH 6.0) to 0.05 mg/mL in terms of BSA, and heat-treated at 60 to 70°C for 10 minutes with a thermal cycler. An enzymatic reaction was carried out using 50 µL of the sample after the heat treatment according to the procedure of Example 1 <1-2>, to thereby calculate an absorbance difference (namely, an absorbance difference between the control sample and the enzyme sample). Similarly, an enzymatic reaction was carried out using an untreated sample, to thereby calculate an absorbance difference. The absorbance difference in the sample after the heat treatment at each temperature was calculated as a relative value to the absorbance difference in the untreated sample which was taken as 100%. This relative value of the absorbance difference was considered as a residual TG activity after the heat treatment at each temperature. As a control, thermostability of a wild-type TG (prepared from Activa(R)) was analyzed via the same procedure.

The results are shown in Table 12. It was confirmed that WT1 to WT5 tend to show thermostability equivalent to or less than that of the wild-type TG (WT). SS1 to SS5 showed improved thermostability compared to SS.

**Table 12**

| | Mutation site | No treatment | 60°C | 65°C | 70°C |
|---|---|---|---|---|---|
| WT | - | 100% | 18% | 3% | 4% |
| WT1 | A81C/C311C | 100% | 21% | 3% | 4% |
| WT2 | E93C/V112C | 100% | 16% | 4% | 5% |
| WT3 | A106C/D213C | 100% | 12% | 7% | 4% |
| WT4 | E107C/Y217C | 100% | 22% | 5% | 4% |
| WT5 | A160C/G228C | 100% | 10% | 2% | 3% |
| SS | D3C/G283C | 100% | 89% | 60% | 6% |
| SS1 | D3C/G283C/A81C/C311C | 100% | 87% | 72% | 36% |
| SS2 | D3C/G283C/E93C/V112C | 100% | 91% | 76% | 23% |
| SS3 | D3C/G283C/A106C/D213C | 100% | 89% | 72% | 13% |
| SS4 | D3C/G283C/E107C/Y217C | 100% | 90% | 78% | 22% |
| SS5 | D3C/G283C/A160C/G228C | 100% | 88% | 67% | 21% |

### Example 5: Analysis of alkaline tolerance of mutant transglutaminases (mutant TGs)

In this Example, alkaline tolerance of mutant TGs were analyzed.

The purified enzymes of the mutant TGs shown in Table 13 (obtained in Example 1) were each diluted with 20 mM MES buffer (pH 6.0), 20 mM Tris-HCl buffer (pH 8.0), or 20 mM CAPS buffer (pH 10.0) to 0.56 U/mL, and treated at 37°C for four hours. The residual TG activity after treatment at each pH was measured by the hydroxamate method. Solution A (50 mM MES, 100 mM NH₂OH, 10 mM glutathione (reduced form), 30 mM CBZ-Gln-Gly (Z-QG), adjusted to pH 6.0 with NaOH) (500 µL) was added in a 1.5 mL tube, and heated at 37°C for 5 min. After 150 µL of an enzyme solution after treatment at each pH was added and the reaction was carried out at 37°C for 30 min, 500 µL of solution B (1N HCl, 4% TCA, 1.67% FeCl₃-6H₂O) was added to stop the reaction. A reaction stop solution (200 µL) was added to a 96-well plate, and the absorbance at 525 nm was measured using a plate reader, to obtain an absorbance of an enzyme sample. As a control, the absorbance was measured for a resulting sample similarly obtained by a reaction using 20 mM MES pH 6.0, to obtain an absorbance of a control sample. A difference between the absorbance of the control sample and the absorbance of the enzyme sample was determined. The absorbance difference in the sample after treatment at each pH was calculated as a relative value to the absorbance difference in the sample after treatment at pH 6.0 which was taken as 100%. This relative value of the absorbance difference was considered as a relative value of the residual TG activity after treatment at each pH to the residual TG activity after treatment at pH 6.0 which was taken as 100%. The alkaline tolerance of a wild-type TG (prepared from Activa(R)) was analyzed via the same procedure.

The results are shown in Table 13. A decrease in the residual TG activity due to alkaline treatment was observed for the wild-type TG, whereas a smaller decrease in the residual TG activity due to alkaline treatment was observed for the mutant TGs than that observed for the wild-type TG. Hence, it was revealed that the alkaline tolerance has been improved in the mutant TGs.

**Table 13 Relative values of residual TG activity after treatment at respective pH**

| | pH 6.0 | pH 8.0 | pH 10.0 |
|---|---|---|---|
| Wild-type TG | 100% | 86% | 49% |
| HT00 | 100% | 87% | 70% |
| HT03 | 100% | 98% | 80% |
| HT10 | 100% | 97% | 80% |
| HT10+E93C/V112C | 100% | 99% | 83% |
| HT10+A160C/G228C | 100% | 97% | 85% |

### Example 6: Preparation and analysis of ice cream using mutant transglutaminase (mutant TG)

In this example, an effect of a mutant TG on physical properties and sensory qualities of ice cream was evaluated.

Ice cream was prepared on the basis of the composition shown in Table 14. As TG, the mutant TG HT-16 (obtained in Example 1) was used. Ice cream prepared without addition of TG was used as a control.

**Table 14**

| Raw material | Mixed amount |
|---|---|
| Cow milk | 27.0% |
| Fresh cream 47% | 15.0% |
| Skim milk (high heat) | 6.4% |
| Granulated sugar | 12.0% |
| Starch syrup | 4.5% |
| Sugared egg yolk | 3.5% |
| Emulsifier | 0.5% |
| Added water | 31.1% |
| Total | 100.0% |

### <Preparation procedure>

Mixing of liquid raw materials;
Mixing of powdery raw materials (at this stage, 0.1% TG HT-16 (100 U/g) was added); Heating and dissolving (75°C, 1 hour);
Emulsification and homogenization (homogenizer, 70°C);
Pasteurization (88°C, 33 sec);
Cooling;
Aging (5°C, 2 hours);
Freezing (-4°C);
Filling up (50% overrun).

A shape retention test (50 mL, at room temperature, 75 minutes, static) was carried out for the prepared ice cream. In addition, a sensory evaluation by expert panels was carried out for the prepared ice cream. In the sensory evaluation, the strength of "richness and fatness" of the control was defined as score 3, and the strength of "richness and fatness" of the TG-added product was evaluated in 0.5-point increments, ranging from score 1 (weak) to score 5 (strong).

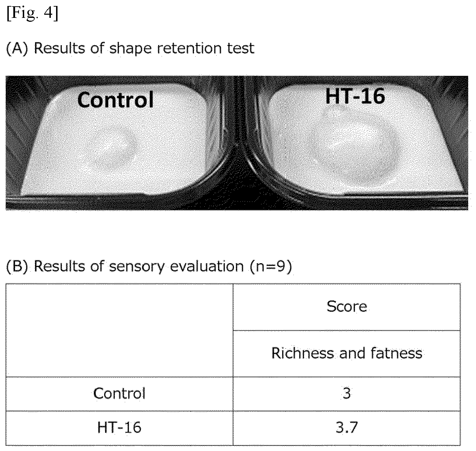

The results are shown in Figure 4. The shape retention and the sensory qualities (specifically, richness and fatness) of ice cream were improved by addition of the mutant TG HT-16.

### Example 7: Preparation and analysis of gummy candy using mutant transglutaminase (mutant TG)

In this example, an effect of a mutant TG on physical properties of gummy candy was evaluated.

Gummy candy was prepared on the basis of the composition shown in Table 15. As TG, the mutant TG HT-16 (obtained in Example 1) was used. Gummy candy prepared without addition of TG was used as a control.

**Table 15**

| Raw material | Mixed amount |
|---|---|
| Gelatin | 4.7% |
| Starch syrup | 44.4% |
| Granulated sugar | 35.0% |
| 50% Citric acid | 1.4% |
| Water | 4.7% |
| Water (for swelling of gelatin) | 9.4% |
| Total | 100.0% |

### <Preparation procedure>

Gelatin was added with water for swelling and allowed to swell for 20 minutes or longer (room temperature);
Starch syrup and granulated sugar were heated and dissolved at 90°C;
The pre-swollen gelatin (the mixture of gelatin and water for swelling) was mixed with water and dissolved in a water bath at 60°C;
The sugar solution at 90°C was combined with the gelatin solution at 60°C and kept warm at 75°C;
50% citric acid solution was added, pH was adjusted to 3.5, and Brix was adjusted to 75;
0.3% TG HT-16 (100 U/g) was added, a reaction was carried out at 60°C, 10 min, 75°C; Molding.

The 80% compressive stress of the prepared gummy candy was measured with a texture analyzer under the following conditions. In addition, a shape retention test (40°C, 2 hours) was carried out for the prepared gummy candy.

### <Measurement conditions with texture analyzer>

Plunger used: Wedge-shaped (20.0 mm in diameter)
Contact Force: 0.5 g
Test Mode: Compression
Test speed: 1.0 mm/sec
Target mode: Strain
Strain: 80%
Trigger type: Auto
Trigger force: 0.5 g

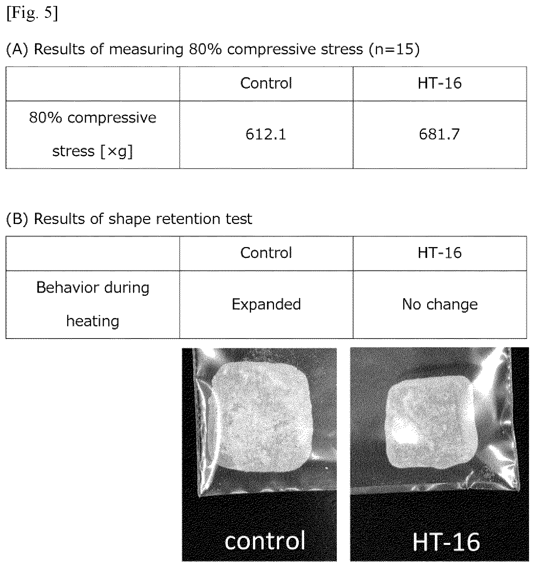

The results are shown in Figure 5. The compressive stress and the shape retention during heating of gummy candy were improved by addition of the mutant TG HT-16.

### Example 8: Preparation and analysis of meat dumplings using mutant transglutaminase (mutant TG)

In this example, an effect of a mutant TG on physical properties of meat dumplings was evaluated.

Three types of meat dumplings having different pH were prepared on the basis of the compositions shown in Table 16. As TG, the wild-type TG (prepared from a strain expressing pPSPTG1) or the mutant TG HT-16 (obtained in Example 1) was used. Meat dumplings prepared without addition of TG were used as a control.

**Table 16**

| Composition 1: Acidic (pH5.97) | |
|---|---|
| Raw material | Mixed amount |
| Minced pork | 89.7% |
| Common salt | 1.3% |
| Water | 9.0% |
| Total | 100% |

| Composition 2: Neutral (pH7.51) | |
|---|---|
| Raw material | Mixed amount |
| Minced pork | 88.5% |
| Common salt | 1.3% |
| Trisodium citrate | 0.8% |
| Calcium oxide | 0.5% |
| Water | 8.8% |
| Total | 100% |

| Composition 3: Alkaline (pH9.35) | |
|---|---|
| Raw material | Mixed amount |
| Minced pork | 87.3% |
| Common salt | 1.3% |
| Trisodium citrate | 1.6% |
| Calcium oxide | 1.0% |
| Water | 8.7% |
| Total | 100% |

### <Preparation procedure>

Mixing of raw materials (half mixed only for water; at this stage, 0.38% TG (the wild-type TG or HT-16; 100 U/g) was added);
Stirring (kitchen mixer, 10 sec);
Adding the remaining water;
Stirring (kitchen mixer, 10 sec);
Molding (length 40 mm, width 90 mm, thickness 20 mm)
Heating (80°C, 30 min);
Deactivation (90°C, 30 min).

The breaking strength of the prepared meat dumplings was measured with a texture analyzer under the following conditions.

### <Measurement conditions with texture analyzer>

Plunger used: Disk type (7 mm diameter)
Contact Force: 0.5 g
Test Mode: Compression
Test speed: 1.0 mm/sec
Target mode: Distance
Distance: 15 mm
Trigger type: Auto
Trigger force: 0.5 g

The results are shown in Table 17. The breaking strength of the meat dumplings was improved at any pH by addition of TGs. In addition, the breaking strength of the meat dumplings was improved at any pH by addition of the mutant TG HT-16 as compared to the case of adding the wild-type TG.

**Table 17**

| Acidic (pH5.97) | | | |
|---|---|---|---|
| | Control | Wild-type TG | HT-16 |
| Breaking strength (g) | 1039.0 | 1161.9 | 1183.2 |

| Neutral (pH7.51) | | | |
|---|---|---|---|
| | Control | Wild-type TG | HT-16 |
| Breaking strength (g) | 1099.8 | 1202.5 | 1282.2 |

| Alkaline (pH9.35) | | | |
|---|---|---|---|
| | Control | Wild-type TG | HT-16 |
| Breaking strength (g) | 747.5 | 782.7 | 791.9 |

### Example 9: Preparation and analysis of steamed egg custards using mutant transglutaminase (mutant TG)

In this example, an effect of a mutant TG on physical properties of steamed egg custards was evaluated.

Two types of steamed egg custards having different pH were prepared on the basis of the compositions shown in Table 18. As TG, the wild-type TG (prepared from a strain expressing pPSPTG1) or the mutant TG HT-16 (obtained in Example 1) was used.

**Table 18**

| Composition 1: Neutral (pH7.6) | |
|---|---|
| Raw material | Mixed amount |
| Whole egg | 100% |
| Total | 100% |

| Composition 2: Acidic (pH5.4) | |
|---|---|
| Raw material | Mixed amount |
| Whole egg | 92.3% |
| Rice vinegar | 7.7% |
| Total | 100% |

### <Preparation procedure>

Mixing of liquid raw materials;
Stirring;
Dispensing (at this stage, 0.49% TG (the wild-type TG or HT-16; 100 U/g) was added);
Dispensing in 20 mL portions;
Heating (75°C, 20 min);
Deactivation (90°C, 20 min);
Cooling (room temperature, 7 hours).

A 15 mm compression test (n=5) was carried out for the prepared steamed egg custards with a texture analyzer under the following conditions.

### <Measurement conditions with texture analyzer>

Plunger used: Cylinder type (10 mm diameter)
Contact Force: 0.5 g
Test Mode: Compression
Test speed: 1.0 mm/sec
Target mode: Distance
Distance: 15 mm
Trigger type: Auto
Trigger force: 0.5 g

The results are shown in Table 19. The compressive strength of the steamed egg custards was improved at any pH by addition of TGs. In addition, the compressive strength of the steamed egg custards was improved at any pH by addition of the mutant TG HT-16 as compared to the case of adding the wild-type TG.

**Table 19**

| Acidic (pH5.4) | | | |
|---|---|---|---|
| | Control | Wild-type TG | HT-16 |
| Compressive strength (g*sec) | 12635.3 | 13406.1 | 14302.4 |

| Neutral (pH7.6) | | | |
|---|---|---|---|
| | Control | Wild-type TG | HT-16 |
| Compressive strength (g*sec) | 12615.6 | 15065.3 | 15561.3 |

### Example 10: Preparation and analysis of jellies using mutant transglutaminase (mutant TG)

In this example, an effect of a mutant TG on physical properties of jellies (namely, gelled gelatin) was evaluated.

Three types of jellies were prepared on the basis of the compositions shown in Table 20. As TG, the wild-type TG (prepared from a strain expressing pPSPTG1) or the mutant TG HT-16 (obtained in Example 1) was used.

**Table 20**

| Composition 1: Alkaline-treated gelatin (pH6.2) | |
|---|---|
| Raw material | Mixed amount |
| Alkaline-treated gelatin of pig skin | 5.3% |
| Water | 94.7% |
| Total | 100% |

| Composition 2: Acid-treated gelatin (pH7.5) | |
|---|---|
| Raw material | Mixed amount |
| Acid-treated gelatin of pig skin | 5.3% |
| 0.1M Phosphate buffer (pH7.5) | 94.7 % |
| Total | 100% |

| Composition 3: Acid-treated gelatin (pH4.8) | |
|---|---|
| Raw material | Mixed amount |
| Acid-treated gelatin of pig skin | 5.3% |
| Water | 94.7% |
| Total | 100% |

### <Preparation procedure>

Adding of 0.3% TG (the wild-type TG or HT-16; 100 U/g) to 15 g of a mixture of raw materials;
Enzyme reaction (static, 70°C, 20 min);
Enzyme deactivation (static, 90°C, 20 min);
Cooling at room temperature for 60 min;
Cooling at 5°C overnight.

A compression test was carried out for the prepared jellies with a texture analyzer under the following conditions, to measure the breaking strength and compression strength.

### <Measurement conditions with texture analyzer>

Plunger used: Cylinder type (12.7 mm diameter)
Contact Force: 0.5 g
Test Mode: Compression
Test speed: 1.0 mm/sec
Target mode: Force
Force: 1019.7 g
Trigger type: Auto
Trigger force: 0.5 g

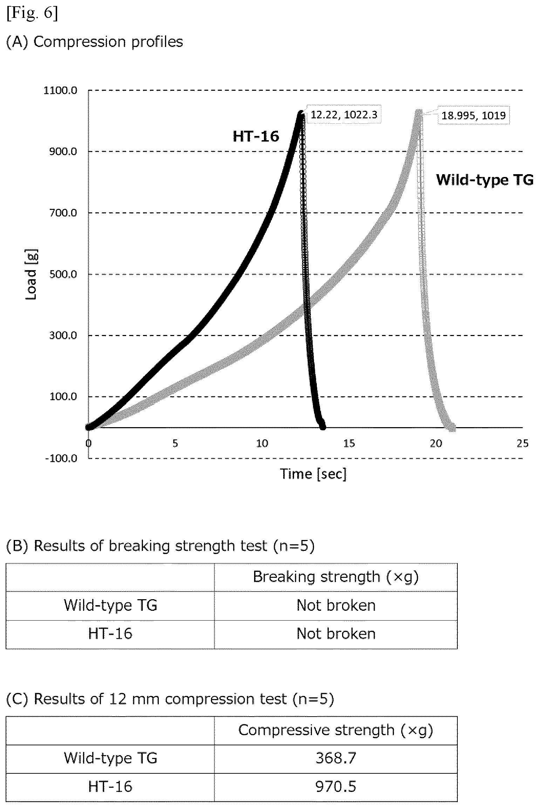

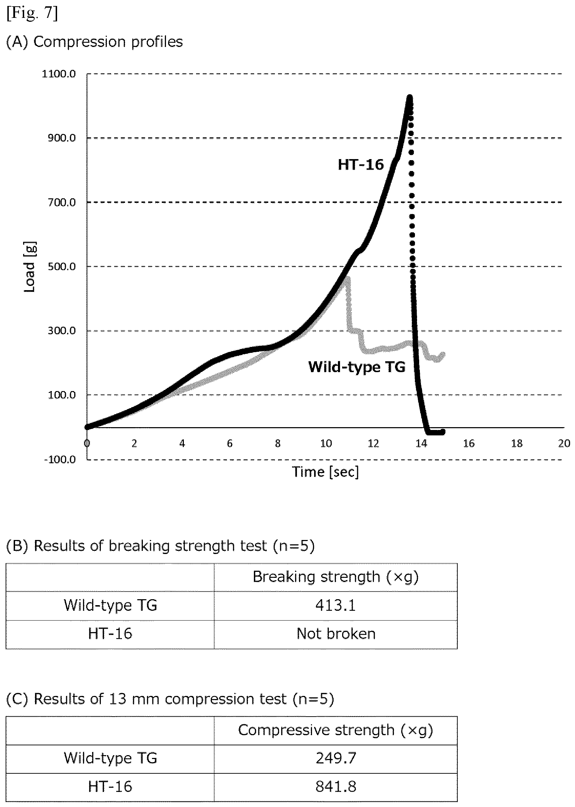

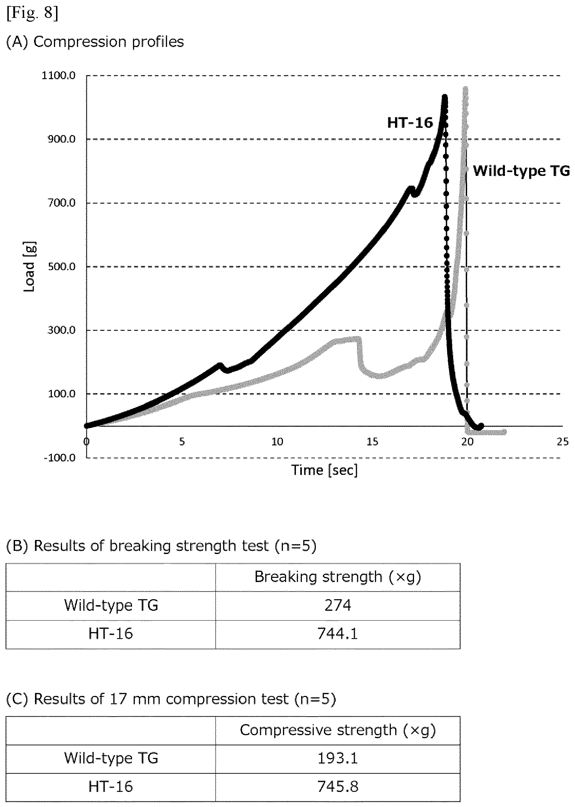

The results of the jellies of Composition 1 to 3 are shown in Figures 6 to 8, respectively. The gel strength was improved and the breaking strength and compression strength (namely, compressive stress) of the jellies were improved for any composition by addition of the mutant TG HT-16 as compared to the case of adding the wild-type TG.

### Example 11: Preparation and analysis of imitation crab using mutant transglutaminase (mutant TG)

In this example, an effect of a mutant TG on physical properties and sensory qualities of imitation crab was evaluated.

Imitation crab was prepared on the basis of the composition shown in Table 21. As TG, the mutant TG HT-16 (obtained in Example 1) was used. Imitation crab prepared without addition of TG was used as a control.

**Table 21**

| Raw material | Mixed amount [%] |
|---|---|
| Frozen minced fish | 54.1 |
| Salt | 1 |
| Sugar | 1.4 |
| MSG | 0.6 |
| Dried egg white | 0.4 |
| Drab extract | 1.4 |
| Starch | 5 |
| Water | 36.6 |
| Total | 100 |

### <Preparation procedure>

Mixing of raw materials other than salt (at this stage, 0.2% TG HT-16 (100 U/g) was added);
Rough grinding;
Salt addition;
Grinding with salt;
Drum heating (94°C);
Molding;
Pasteurization (90°C, 20 min).

The breaking strength of the prepared imitation crab was measured with a texture analyzer. In addition, a sensory evaluation by expert panels was carried out for the prepared imitation crab. In the sensory evaluation, the strength of "hardness" and "suppleness and elongation" of the control was defined as score 3, and the strength of "hardness" and "suppleness and elongation" of the TG-added product was evaluated in 0.5-point increments, ranging from score 1 (weak) to score 5 (strong).

The results are shown in Table 22. The breaking strength and the sensory qualities (specifically, "hardness" and "suppleness and elongation") of imitation crab were improved by addition of the mutant TG HT-16.

**Table 22**

| (A) Results of measuring breaking strength (n=15) | | | |
|---|---|---|---|
| | Breaking strength (×g) | | |
| Control | 817 | | |
| HT-16 | 1145 | | |

| (B) Results of sensory evaluation (n=9) | | | |
|---|---|---|---|
| | Score | | |
| | Hardness | Supplen ness and elongation | |
| Control | 3 | 3 | |
| HT-16 | 3.8 | 3.6 | |

### Example 12: Preparation and analysis of deep-fried fish paste using mutant transglutaminase (mutant TG)

In this example, an effect of a mutant TG on physical properties of deep-fried fish paste was evaluated.

Deep-fried fish paste was prepared on the basis of the composition shown in Table 23. As TG, the mutant TG HT-16 (obtained in Example 1) was used. Deep-fried fish paste prepared without addition of TG was used as a control.

**Table 23**

| | Mixed amount [%] | |
|---|---|---|
| Raw material | Control | HT-16 |
| Frozen minced fish | 40 | 40 |
| Soybean protein | 3 | 3 |
| Salt | 1 | 1 |
| Sugar | 1.3 | 1.3 |
| Dried egg white | 1 | 1 |
| Starch | 12 | 12 |
| Glucose | 1.7 | 1.7 |
| Sorbitol | 6 | 6 |
| MSG | 1 | 1 |
| Water | 33 | 33 |
| HT-16 (100 U/g) | 0 | 0.2 |
| Total | 100 | 100.2 |

### <Preparation procedure>

Mixing of raw materials other than salt (at this stage, 0.2% TG HT-16 (100 U/g) was added);
Rough grinding;
Salt addition;
Grinding with salt;
Molding (10 mm thick deep-fried fish paste);
Cooking with oil (1) (135°C, 120 sec);
Cooking with oil (2) (165°C, 90 sec);
Cooling.

The breaking strength of the prepared deep-fried fish paste was measured with a texture analyzer.

The results are shown in Table 24. The breaking strength of deep-fried fish paste was improved by addition of the mutant TG HT-16.

**Table 24 Results of measuring breaking strength (n=15)**

| | Control | HT-16 |
|---|---|---|
| Breaking strength (×g) | 792.3 | 900.1 |

### <Explanation of Sequence Listing>

### SEQ ID NOS:

1: Nucleotide sequence of a part of the TG gene of *Streptoverticillium mobaraense,* the part encoding the mature TG
2: Amino acid sequence of the mature TG of *Streptoverticillium mobaraense*
3: Nucleotide sequence of the TG gene of *Streptoverticillium mobaraense*
4: Amino acid sequence of TG of *Streptoverticillium mobaraense* comprising the pro-structure moiety
   5 to 114: Primers

### Industrial Applicability

According to the present invention, a mutant TG having high thermostability and/or high pH stability can be provided. The mutant TG having high thermostability and/or high pH stability can be useful for, for example, modification or production of a food such as processed cheese.

## Claims

1. A method for producing a food, the method comprising:
a step of treating a food raw material with a mutant transglutaminase under heating conditions, acidic condition, or alkaline conditions,
wherein the mutant transglutaminase is a protein having a specific mutation in the amino acid sequence of a wild-type transglutaminase, and having a transglutaminase activity, and
wherein the specific mutation is a mutation of improving thermostability and/or pH stability.

2. The method according to claim 1, wherein the specific mutation comprises the mutation (A) and/or the mutation (B) shown below:
the mutation (A): mutation(s) at one or more amino acid residues selected from the followings:
G275, D1, Y24, R48, S101, G102, N139, D142, L147, K152, G157, R167, N176, K181, E182, H188, D189, R208, T245, S246, G250, S284, H289, G301, and K327;
the mutation (B): a mutation of introducing a disulfide bond.

3. The method according to claim 2, wherein the mutation (A) comprises mutation(s) at one or more amino acid residues selected from the followings:
G275, S101, G157, R208, and G250.

4. The method according to claim 2 or 3, wherein the mutation (A) comprises one or more mutations selected from the followings:
G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.

5. The method according to any one of claims 2 to 4, wherein the mutation (A) comprises one or more mutations selected from the followings:
G275A, S101P, G157(A, R, S), R208E, and G250(N, R, S).

6. The method according to any one of claims 2 to 5, wherein the mutation (A) comprises any one of the following mutations:
S101P/G157(A, R, S)/R208E/G250(N, R, S)/G275A, S101P/G157(A, R, S)/G250(N, R, S), and S101P/G157(A, R, S)/R208E/G250(N, R, S).

7. The method according to any one of claims 2 to 6, wherein the mutation (A) comprises any one of the following mutations:
S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.

8. The method according to any one of claims 2 to 7, wherein the mutation (A) comprises any one of the following mutations:
S101P/G157R/R208E/G250S/G275A and
S101P/G157S/R208E/G250R/G275A.

9. The method according to any one of claims 2 to 8, wherein the mutation (B) comprises one or more mutations selected from the followings:
D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.

10. The method according to any one of claims 2 to 9, wherein the mutation (B) is mutations of introducing two or more disulfide bonds.

11. The method according to any one of claims 2 to 10, wherein the mutation (B) is mutations of introducing three or more disulfide bonds.

12. The method according to any one of claims 2 to 11, wherein the mutation (B) comprises D3C/G283C.

13. The method according to any one of claims 2 to 12, wherein the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.

14. The method according to any one of claims 2 to 13, wherein the mutation (B) comprises any one of the following mutations:
D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.

15. The method according to any one of claims 2 to 14, wherein the specific mutation comprises the mutation (A) and the mutation (B).

16. The method according to any one of claims 1 to 15, wherein the residual activity after heat-treating the mutant transglutaminase at 65°C, pH6.0 for 10 minutes is 15% or more.

17. The method according to any one of claims 1 to 16, wherein the residual activity after heat-treating the mutant transglutaminase at 75°C, pH6.0 for 10 minutes is 10% or more.

18. The method according to any one of claims 1 to 17, wherein the residual activity after treating the mutant transglutaminase at pH4.0, 37°C for four hours is 60% or more in term of the relative value to the residual activity after treating the mutant TG at pH6.0, 37°C for four hours which is taken as 100%.

19. The method according to any one of claims 1 to 18, wherein the wild-type transglutaminase is a protein having an amino acid sequence of a mature transglutaminase of a *Streptomyces* bacterium.

20. The method according to claim 19, wherein the *Streptomyces* bacterium is *Streptomyces mobaraensis.*

21. The method according to any one of claims 1 to 20, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

22. The method according to any one of claims 1 to 21, which satisfies any one of (1) to (3) shown below:
(1) the step is carried out under the heating conditions, and the food is a milk processed food, egg processed food, plant processed food, meat processed food, seafood processed food, or gelatin processed food;
(2) the step is carried out under the acidic conditions, and the food is a cereal, potato, bean, nut, meat, seafood, dairy product, fat or oil, seasoning, beverage, fruit juice, or a processed product thereof;
(3) the step is carried out under the alkaline conditions, and the food is a plant beverage, a seaweed extract, or a processed product thereof.

23. The method according to any one of claims 1 to 22, which satisfies any one of (1) to (3) shown below:
(1) the step is carried out under the heating conditions, and the food is processed cheese, pre-incubated yogurt, egg omelet, steamed egg custard, egg tofu, tofu, yuba, noodle, bread, ham, sausage, hamburger steak, ice cream, wheat dough, jelly, gummy, margarine, or paste product;
(2) the step is carried out under the acidic conditions, and the food is white rice, brown rice, vinegared rice, oatmeal, rice flour, wheat flour, buckwheat flour, bran, rice cake, gyuhi, bread, noodle, fu, buckwheat noodle, white potato, soy milk, chocolate, fried tofu, pea protein, meat, gelatin, shrimp, crab, adductor, herring roe, yogurt, butter, cheese, ice cream, mayonnaise, ketchup, mustard, soy sauce, miso, coffee-based drink, nutritional drink, fruit juice drink, cocoa, beer, sake lee, fruit juice gummy, or a processed product thereof;
(3) the step is carried out under the alkaline conditions, and the food is soy milk, kelp soup stock, or a processed product thereof.

24. The method according to any one of claims 1 to 23, wherein a heating temperature during the step is 60°C or higher.

25. A composition for producing a food, the composition comprising a mutant transglutaminase,
wherein the food is a food that is heated during production or a food having low pH or high pH,
wherein the mutant transglutaminase is a protein having a specific mutation in the amino acid sequence of a wild-type transglutaminase, and having a transglutaminase activity, and
wherein the specific mutation is a mutation of improving thermostability and/or pH stability.

26. The composition according to claim 25, wherein the specific mutation comprises the mutation (A) and/or the mutation (B) shown below:
the mutation (A): mutation(s) at one or more amino acid residues selected from the followings:
G275, D1, Y24, R48, S101, G102, N139, D142, L147, K152, G157, R167, N176, K181, E182, H188, D189, R208, T245, S246, G250, S284, H289, G301, and K327;
the mutation (B): a mutation of introducing a disulfide bond.

27. The composition according to claim 26, wherein the mutation (A) comprises mutation(s) at one or more amino acid residues selected from the followings:
G275, S101, G157, R208, and G250.

28. The composition according to claim 26 or 27, wherein the mutation (A) comprises one or more mutations selected from the followings:
G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.

29. The composition according to any one of claims 26 to 28, wherein the mutation (A) comprises one or more mutations selected from the followings:
G275A, S101P, G157(A, R, S), R208E, and G250(N, R, S).

30. The composition according to any one of claims 26 to 29, wherein the mutation (A) comprises any one of the following mutations:
S101P/G157(A, R, S)/R208E/G250(N, R, S)/G275A, S101P/G157(A, R, S)/G250(N, R, S), and S101P/G157(A, R, S)/R208E/G250(N, R, S).

31. The composition according to any one of claims 26 to 30, wherein the mutation (A) comprises any one of the following mutations:
S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.

32. The composition according to any one of claims 26 to 31, wherein the mutation (A) comprises any one of the following mutations:
S101P/G157R/R208E/G250S/G275A and
S101P/G157S/R208E/G250R/G275A.

33. The composition according to any one of claims 26 to 32, wherein the mutation (B) comprises one or more mutations selected from the followings:
D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.

34. The composition according to any one of claims 26 to 33, wherein the mutation (B) is mutations of introducing two or more disulfide bonds.

35. The composition according to any one of claims 26 to 34, wherein the mutation (B) is mutations of introducing three or more disulfide bonds.

36. The composition according to any one of claims 26 to 35, wherein the mutation (B) comprises D3C/G283C.

37. The composition according to any one of claims 26 to 36, wherein the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.

38. The composition according to any one of claims 26 to 37, wherein the mutation (B) comprises any one of the following mutations:
D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.

39. The composition according to any one of claims 26 to 38, wherein the specific mutation comprises the mutation (A) and the mutation (B).

40. The composition according to any one of claims 25 to 39, wherein the residual activity after heat-treating the mutant transglutaminase at 65°C, pH6.0 for 10 minutes is 15% or more.

41. The composition according to any one of claims 25 to 40, wherein the residual activity after heat-treating the mutant transglutaminase at 75°C, pH6.0 for 10 minutes is 10% or more.

42. The composition according to any one of claims 25 to 41, wherein the residual activity after treating the mutant transglutaminase at pH4.0, 37°C for four hours is 60% or more in term of the relative value to the residual activity after treating the mutant TG at pH6.0, 37°C for four hours which is taken as 100%.

43. The composition according to any one of claims 25 to 42, wherein the wild-type transglutaminase is a protein having an amino acid sequence of a mature transglutaminase of a *Streptomyces* bacterium.

44. The composition according to claim 43, wherein the *Streptomyces* bacterium is *Streptomyces mobaraensis.*

45. The composition according to any one of claims 25 to 44, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

46. The composition according to any one of claims 25 to 45, which satisfies any one of (1) to (3) shown below:
(1) the step is carried out under the heating conditions, and the food is a milk processed food, egg processed food, plant processed food, meat processed food, seafood processed food, or gelatin processed food;
(2) the step is carried out under the acidic conditions, and the food is a cereal, potato, bean, nut, meat, seafood, dairy product, fat or oil, seasoning, beverage, fruit juice, or a processed product thereof;
(3) the step is carried out under the alkaline conditions, and the food is a plant beverage, a seaweed extract, or a processed product thereof.

47. The composition according to any one of claims 25 to 46, which satisfies any one of (1) to (3) shown below:
(1) the step is carried out under the heating conditions, and the food is processed cheese, pre-incubated yogurt, egg omelet, steamed egg custard, egg tofu, tofu, yuba, noodle, bread, ham, sausage, hamburger steak, ice cream, wheat dough, jelly, gummy, margarine, or paste product;
(2) the step is carried out under the acidic conditions, and the food is white rice, brown rice, vinegared rice, oatmeal, rice flour, wheat flour, buckwheat flour, bran, rice cake, gyuhi, bread, noodle, fu, buckwheat noodle, white potato, soy milk, chocolate, fried tofu, pea protein, meat, gelatin, shrimp, crab, adductor, herring roe, yogurt, butter, cheese, ice cream, mayonnaise, ketchup, mustard, soy sauce, miso, coffee-based drink, nutritional drink, fruit juice drink, cocoa, beer, sake lee, fruit juice gummy, or a processed product thereof;
(3) the step is carried out under the alkaline conditions, and the food is soy milk, kelp soup stock, or a processed product thereof.

48. The composition according to any one of claims 25 to 47, wherein a heating temperature during the production is 60°C or higher.

49. A mutant transglutaminase, having a specific mutation in the amino acid sequence of a wild-type transglutaminase, and having a transglutaminase activity,
wherein the specific mutation is a mutation of improving thermostability and/or pH stability,
wherein the specific mutation comprises the mutation (A) and/or the mutation (B) shown below:
the mutation (A): mutation(s) at one or more amino acid residues selected from the followings:
G275, D1, Y24, R48, S101, G102, N139, D142, L147, K152, G157, R167, N176, K181, E182, H188, D189, R208, T245, S246, G250, S284, H289, G301, and K327;
the mutation (B): a mutation of introducing a disulfide bond, and
wherein the mutant transglutaminase satisfies (B1) and/or (A1) shown below: (A1) the specific mutation comprises the mutation (A), and the mutation (A) comprises mutation(s) at one or more amino acid residues selected from the followings:
D1, R48, G102, N139, D142, L147, K152, R167, N176, K181, E182, D189, S246, H289, G301, and K327,
provided that the mutation at R48 is R48I when the mutation at R48 is solely selected, and
provided that the mutation at S246 is S246N when the mutation at S246 is solely selected;
(B1) the specific mutation comprises the mutation (B), and the mutation (B) comprises a combination of D3C/G283C and one or more mutations selected from E93C/V112C, A81C/V311C, A106C/D213C, E107C/Y217C, A160C/G228C, S84C/K121C, R79C/P169C, A113C/P220C, E119C/S299C, and R89C/S116C.

50. The mutant transglutaminase according to claim 49, wherein the mutation (A) comprises mutation(s) at one or more amino acid residues selected from the followings:
G275, S101, G157, R208, and G250.

51. The mutant transglutaminase according to claim 49 or 50, wherein the mutation (A) comprises one or more mutations selected from the followings:
G275A, D1F, Y24(G, N), R48(I, K), S101(G, A, V, I, P, F, N, Q, Y, K, R, E), G102N, N139S, D142A, L147W, K152T, G157(A, V, I, S, N, K, R, H, D, E), R167G, N176D, K181R, E182D, H188Y, D189I, R208(L, A, E), T245A, S246(K, N, R), G250(A, V, L, M, P, F, W, S, T, N, Q, Y, K, R, H, D), S284P, H289I, G301W, and K327F.

52. The mutant transglutaminase according to any one of claims 49 to 51, wherein the mutation (A) comprises one or more mutations selected from the followings:
G275A, S101P, G157(A, R, S), R208E, and G250(N, R, S).

53. The mutant transglutaminase according to any one of claims 49 to 52, wherein the mutation (A) comprises any one of the following mutations:
S101P/G157(A, R, S)/R208E/G250(N, R, S)/G275A, S101P/G157(A, R, S)/G250(N, R, S), and S101P/G157(A, R, S)/R208E/G250(N, R, S).

54. The mutant transglutaminase according to any one of claims 49 to 53, wherein the mutation (A) comprises any one of the following mutations:
S101P/G157R/R208E/G250S/G275A, S101P/G157S/R208E/G250R/G275A, S101P/G157S/R208E/G250S, S101P/G157A/R208E/G250S, S101P/G157R/R208E/G250S, and S101P/G157R/R208E/G250N.

55. The mutant transglutaminase according to any one of claims 49 to 54, wherein the mutation (A) comprises any one of the following mutations:
S101P/G157R/R208E/G250S/G275A and
S101P/G157S/R208E/G250R/G275A.

56. The mutant transglutaminase according to any one of claims 49 to 55, wherein the mutation (B) comprises one or more mutations selected from the followings:
D3C/G283C, A81C/V311C, E93C/V112C, A106C/D213C, E107C/Y217C, A160C/G228C, S2C/N282C, S2C/G283C, T7C/E58C, P17C/W330C, D46C/S318C, S84C/K121C, R79C/P169C, A113C/P220C, E119C/S299C, and R89C/S116C.

57. The mutant transglutaminase according to any one of claims 49 to 56, wherein the mutation (B) is three or more mutations of introducing disulfide bonds.

58. The mutant transglutaminase according to any one of claims 49 to 57, wherein the mutation (B) comprises any one of the following mutations:
D3C/G283C/E93C/V112C, D3C/G283C/A81C/V311C, D3C/G283C/A106C/D213C, D3C/G283C/E107C/Y217C, D3C/G283C/A160C/G228C, D3C/G283C/S84C/K121C, D3C/G283C/R79C/P169C, D3C/G283C/A113C/P220C, D3C/G283C/E119C/S299C, D3C/G283C/R89C/S116C, D3C/G283C/E93C/V112C/E107C/Y217C, D3C/G283C/E93C/V112C/A113C/P220C, D3C/G283C/E93C/V112C/E119C/S299C, D3C/G283C/E107C/Y217C/S84C/K121C, and D3C/G283C/S84C/K121C/A113C/P220C.

59. The mutant transglutaminase according to any one of claims 49 to 58, wherein the specific mutation comprises the mutation (A) and the mutation (B).

60. The mutant transglutaminase according to any one of claims 49 to 59, wherein the residual activity after heat-treating the mutant transglutaminase at 65°C, pH6.0 for 10 minutes is 15% or more.

61. The mutant transglutaminase according to any one of claims 49 to 60, wherein the residual activity after heat-treating the mutant transglutaminase at 75°C, pH6.0 for 10 minutes is 10% or more.

62. The mutant transglutaminase according to any one of claims 49 to 61, wherein the residual activity after treating the mutant transglutaminase at pH4.0, 37°C for four hours is 60% or more in term of the relative value to the residual activity after treating the mutant TG at pH6.0, 37°C for four hours which is taken as 100%.

63. The mutant transglutaminase according to any one of claims 49 to 62, wherein the wild-type transglutaminase is a protein having an amino acid sequence of a mature transglutaminase of a *Streptomyces* bacterium.

64. The mutant transglutaminase according to claim 63, wherein the *Streptomyces* bacterium is *Streptomyces mobaraensis.*

65. The mutant transglutaminase according to any one of claims 49 to 64, wherein the wild-type transglutaminase is any of the following proteins:
(a) a protein comprising the amino acid sequence of SEQ ID NO: 2;
(b) a protein comprising the amino acid sequence of SEQ ID NO: 2 but including substitution, deletion, insertion, and/or addition of 1 to 10 amino acid residues; and
(c) a protein comprising an amino acid sequence having an identity of 90% or higher to the amino acid sequence of SEQ ID NO: 2.

66. A gene encoding the mutant transglutaminase according to any one of claims 49 to 65.

67. A vector comprising the gene according to claim 66.

68. A microorganism having the gene according to claim 66.

69. The microorganism according to claim 68, which is a bacterium or a yeast.

70. The microorganism according to claim 68 or 69, which is a coryneform bacterium or a bacterium of the family *Enterobacteriaceae.*

71. The microorganism according to any one of claims 68 to 70, which is a *Corynebacterium* bacterium or an *Escherichia* bacterium.

72. The microorganism according to any one of claims 68 to 71, which is *Corynebacterium glutamicum* or *Escherichia coli.*
